# EUROPEAN PATENT APPLICATION

(11) **EP 4 640 190 A2**
(43) Date of publication of application: **29.10.2025**
(21) Application number: 25199406.7
(22) Date of filing: 06.09.2021
(51) Int. Cl.: A61F 2/90

(54) **STENT**

(30) Priority: 20.10.2020 JP 2020176275
(62) Divisional of application: 21882454.8
(71) Applicant: T.G. Medical Inc., Tokyo 103-0023 (JP)
(72) Inventor: SHOBAYASHI, Yasuhiro, Tokyo, 103-0023 (JP); MIKI, Kohei, Tokyo, 103-0023 (JP)
(74) Representative: Ström & Gulliksson AB

(57) **Abstract**

A stent 1 which is inserted into a catheter and extruded from the catheter into a blood vessel to dilate a blood vessel, wherein the stent is equipped with a first stent body 10 in which a plurality of first cells comprising struts arranged in a frame shape are spread in the circumferential direction and are contiguous in the central axial direction and a second stent body 20, interpolated into the first stent body, in which a plurality of second cells comprising struts arranged in a frame shape are spread in the circumferential direction and are contiguous in the central axial direction, and, in a state in which the second stent body 20 is interpolated into the first stent body 10, the intersecting portions of the second cells are arranged in the hole portions of the first cells and the first stent body 10 and the second stent body 20 are not connected to each other in the radial direction.

## Description

### TECHNICAL FIELD

The present invention relates to a stent used to expand a lumen.

### BACKGROUND ART

Typically, treatment has been performed, in which, e.g., a cardiovascular, cerebral-vascular, or peripheral-vascular lumen narrowed or occluded with, e.g., plaque and became ischemic accordingly is expanded in order to ensure the patency of a lesion area. For example, a catheter treatment has been known, in which a stent or a balloon sheathed in a catheter is deployed in a lesion area. As one example of the stent used for such catheter treatment, a stent provided with a plurality of struts which extends radially from a center axis has been proposed (see Patent Document 1).

Patent Document 1: US Patent No. 10390982

### DISCLOSURE OF THE INVENTION

### Problems to be Solved by the Invention

In the case of using an indwelling stent for expanding a vascular lumen, there is a probability that restenosis or reocclusion occurs in a blood vessel after implantation of the stent or a complication such as a thrombosis occurs. On the other hand, in the case of using a balloon for expanding a vascular lumen, a blood vessel is temporarily closed, and for this reason, there is a probability that infarction particularly in a distal side blood vessel occurs. Further, there is a concern regarding, e.g., limitation on an expansion time and a remaining narrowed lesion area and restenosis after treatment. In addition, since the blood vessel expanded by the balloon is in a linear shape, there is a probability that a hemorrhagic complication due to, e.g., blood vessel damage or rupture or infarction of a penetrating branch of a peripheral blood vessel occurs.

In the case of a recovery stent such as the stent of Patent Document 1, the stent is recovered after having been temporarily implanted in a blood vessel, so that the various complication risks as described above can be reduced while the patency of the blood vessel is ensured. However, if the surface area of the stent is increased in order to more uniformly expand the narrowed blood vessel, the bending stiffness of the stent becomes too high, leading to poor shape followability to a vascular structure. If the surface area (the area excluding the area of the cell holes) of the stent is increased, the volume of the stent increases, and for this reason, it is difficult to sheathe the narrowed stent in a thin catheter. Since the stent of Patent Document 1 includes the plurality of struts, it is assumed that shape followability and diameter reducibility are significantly degraded if the surface area is merely increased.

An object of the present invention is to provide a stent having a large surface area and having excellent shape followability to a vascular structure and excellent diameter reducibility.

### Means for Solving the Problems

The present invention relates to a stent that is inserted into a catheter and pushed out of the catheter in a blood vessel to expand the blood vessel. The stent includes a first stent body configured such that a plurality of first cells including struts arranged in a frame shape spreads in a circumferential direction and is continuously arranged in a center axis direction, and a second stent body configured such that a plurality of second cells including struts arranged in a frame shape spreads in a circumferential direction and is continuously arranged in a center axis direction and inserted into the first stent body. In a state in which the second stent body is inserted into the first stent body, an intersection between the second cells is arranged in a hole of each first cell. The first stent body and the second stent body are not coupled to each other in a radial direction.

In the state in which the second stent body is inserted into the first stent body, the second stent body may press the first stent body outward in the radial direction.

In a configuration in which the intersection between the second cells is arranged in the hole of each first cell, one intersection between the second cells may be arranged in one hole of each first cell.

In the state in which the second stent body is inserted into the first stent body, the percentage of a non-hole portion per unit surface area in a portion where the first stent body and the second stent body overlap with each other may be 5 to 50%.

Each first cell may include, in an annular direction inclined with respect to the circumferential direction, a pair of first struts and one first strut arranged with a clearance from the pair of first struts, and each second cell may include, in an annular direction inclined with respect to the circumferential direction, a pair of second struts and one second strut arranged with a clearance from the pair of second struts.

Adjacent ones of the plurality of first cells may be connected to each other at a substantially S-shaped first intersection in the annular direction inclined with respect to the circumferential direction, and adjacent ones of the plurality of second cells may be connected to each other at a substantially S-shaped second intersection in the annular direction inclined with respect to the circumferential direction.

The annular direction in which the plurality of first cells is connected at the first intersections and the annular direction in which the plurality of second cells is connected at the second intersections may be symmetrical with respect to a line along the radial direction.

A proximal side end portion of the first stent body and a proximal side end portion of the second stent body may be connected at different positions in the axial direction of a push wire.

The stent may further include a coating film between the first stent body and the second stent body.

A strand having a high radiopacity may be wound around at least one of the first stent body or the second stent body in a spiral shape.

### Effects of the Invention

According to the present invention, a stent can be provided, which has a large surface area and having excellent shape followability to a vascular structure and excellent diameter reducibility.

### BRIEF DESCRIPTION OF THE DRAWINGS

FIG. 1 is a schematic side view of a stent 1 of a first embodiment;
FIG. 2 is a schematic perspective view of the stent 1 shown in FIG. 1;
FIG. 3A is a development view showing a state in which a first stent body 10 is virtually opened in a planar shape;
FIG. 3B is a development view showing a state in which a second stent body 20 is virtually opened in a planar shape;
FIG. 3C is a development view showing a state in which the stent 1 of the first embodiment is virtually opened in a planar shape;
FIG. 4A is a view for describing the outer diameter D1 of the simple first stent body 10;
FIG. 4B is a view for describing the outer diameter D2 of the simple second stent body 20;
FIG. 5 is a view for describing steps of inserting the second stent body 20 into the first stent body 10;
FIG. 6 is a sectional view along an s1-s1 line of FIG. 1;
FIG. 7 is a view for describing an internal state when the stent 1 is bent;
FIG. 8A is a development view showing a state in which a first stent body 110 of a second embodiment is virtually opened in a planar shape;
FIG. 8B is a development view showing a state in which a second stent body 120 of the second embodiment is virtually opened in a planar shape;
FIG. 8C is a development view showing a state in which a stent 1A of the second embodiment is virtually opened in a planar shape;
FIG. 9A is a development view showing a state in which a first stent body 210 of a third embodiment is virtually opened in a planar shape;
FIG. 9B is a development view showing a state in which a second stent body 220 of the third embodiment is virtually opened in a planar shape;
FIG. 9C is a development view showing a state in which a stent 1B of the third embodiment is virtually opened in a planar shape;
FIG. 10A is a sectional view when the stent 1A of the second embodiment is expanded in a blood vessel;
FIG. 10B is a sectional view when the stent 1B of the third embodiment is expanded in a blood vessel;
FIG. 11 is a schematic perspective view of a stent 1C of a fourth embodiment;
FIG. 12A is a development view showing a state in which a first stent body 310 of the fourth embodiment is virtually opened in a planar shape;
FIG. 12B is a development view showing a state in which a second stent body 320 of the fourth embodiment is virtually opened in a planar shape;
FIG. 12C is a development view showing a state in which the stent 1C of the fourth embodiment is virtually opened in a planar shape;
FIG. 13 is a schematic perspective view of a stent 1D of a fifth embodiment;
FIG. 14A is a development view showing a state in which a first stent body 410 of the fifth embodiment is virtually opened in a planar shape;
FIG. 14B is a development view showing a state in which a second stent body 420 of the fifth embodiment is virtually opened in a planar shape;
FIG. 14C is a development view showing a state in which the stent 1D of the fifth embodiment is virtually opened in a planar shape;
FIG. 15 is a schematic perspective view of a stent 1E of a sixth embodiment;
FIG. 16A is a development view showing a state in which a first stent body 510 of the sixth embodiment is virtually opened in a planar shape;
FIG. 16B is a development view showing a state in which a second stent body 520 of the sixth embodiment is virtually opened in a planar shape;
FIG. 16C is a development view showing a state in which the stent 1E of the sixth embodiment is virtually opened in a planar shape;
FIG. 17A is a side view schematically showing a configuration in which a proximal side end portion of the stent 1 and a push wire 2 are connected in a first connection form;
FIG. 17B is a sectional view along an s4-s4 line of FIG. 17A;
FIG. 18A is a side view schematically showing a configuration in which the proximal side end portion of the stent 1 and the push wire 2 are connected in a second connection form;
FIG. 18B is a sectional view along an s5-s5 line of FIG. 18A;
FIG. 18C is a sectional view along an s6-s6 line of FIG. 18A;
FIG. 19A is a side view schematically showing a configuration in which the proximal side end portion of the stent 1 and the push wire 2 are connected in a third connection form;
FIG. 19B is a sectional view along an s7-s7 line of FIG. 19A;
FIG. 19C is a sectional view along an s8-s8 line of FIG. 19A;
FIG. 20 is a side view schematically showing a configuration in which a distal side end portion of the stent 1 and a distal end shaft 3 are connected in the first connection form;
FIG. 21 is a side view schematically showing another configuration in which the distal side end portion of the stent 1 and the distal end shaft 3 are connected in the first connection form;
FIG. 22 is a side view schematically showing a configuration in which the distal side end portion of the stent 1 and the distal end shaft 3 are connected in a fourth connection form;
FIG. 23 is a side view schematically showing another configuration of the stent 1 on the distal side thereof;
FIG. 24 is a schematic side view of a stent 1F of an eleventh embodiment;
FIG. 25 is a sectional view along an s9-s9 line of FIG. 24;
FIG. 26 is a schematic view showing an example where a strand 31 having a high radiopacity is sparsely wound around a strut 11 of the first stent body 10;
FIG. 27 is a schematic view showing an example where the strand 31 having the high radiopacity is densely wound around the strut 11 of the first stent body 10;
FIG. 28 is a schematic view showing an example where the strand 31 having the high radiopacity is wound around the stent 1 in a first form;
FIG. 29 is a schematic view showing an example where the strand 31 having the high radiopacity is wound around the stent 1 in a second form;
FIG. 30 is a schematic perspective view of a stent 1G of a thirteenth embodiment;
FIG. 31A is a development view showing a state in which part of a first stent body 610 of the thirteenth embodiment is virtually opened in a planar shape;
FIG. 31B is a development view showing a state in which part of a second stent body 620 of the thirteenth embodiment is virtually opened in a planar shape;
FIG. 31C is a development view showing a state in which part of the stent 1G of the thirteenth embodiment is virtually opened in a planar shape;
FIG. 32A is a development view showing a state in which a first stent body 310 of a fourteenth embodiment is virtually opened in a planar **shape;**
**FIG.** 32B is a development view showing a state in which a second stent body 320 of the fourteenth embodiment is virtually opened in a planar **shape;**
**FIG.** 32C is a development view showing a state in which a stent 1H of the fourteenth embodiment is virtually opened in a planar **shape;**
**FIG.** 33A is a development view showing a state in which a first stent body 310 of a fifteenth embodiment is virtually opened in a planar **shape;**
FIG. 33B is a development view showing a state in which a second stent body 320 of the fifteenth embodiment is virtually opened in a planar shape;
FIG. 33C is a development view showing a state in which a stent 1J of the fifteenth embodiment is virtually opened in a planar shape;
FIG. 34A is a development view showing a state in which a first stent body 310 of a sixteenth embodiment is virtually opened in a planar shape;
FIG. 34B is a development view showing a state in which a second stent body 320 of the sixteenth embodiment is virtually opened in a planar shape; and
FIG. 34C is a development view showing a state in which a stent 1K of the sixteenth embodiment is virtually opened in a planar shape.

### PREFERRED MODE FOR CARRYING OUT THE INVENTION

Hereinafter, embodiments of a stent according to the present invention will be described. Note that any of the drawings attached to the present specification shows a schematic view and the shape, scale, longitudinal-lateral dimensional ratio, etc. of each portion are changed or exaggerated as compared to actual shape, scale, longitudinal-lateral dimensional ratio, etc. for the sake of easy understanding of the drawings. Moreover, in the drawings, hatching showing the cross-section of members has been omitted where appropriate. In the present specification etc., terms specifying shapes, geometric conditions, and the degrees thereof, such as "parallel" and "direction", include not only exact meanings of these terms, but also ranges taken as being substantially parallel and being substantially in a direction. In description in the present specification etc., in an axial direction (a center axis direction) LD, a proximal side close to a practitioner will be referred to as a side LD1, and a distal side distant from the practitioner will be referred to as a side LD2. A direction perpendicular to the axial direction LD will be referred to as a radial direction RD. Moreover, in description in the present specification etc., a direction in which cells spread will be referred to as a circumferential direction (a circumferential direction OD). The circumferential direction includes not only the radial direction RD, but also a direction inclined with respect to the radial direction RD.

### (First Embodiment)

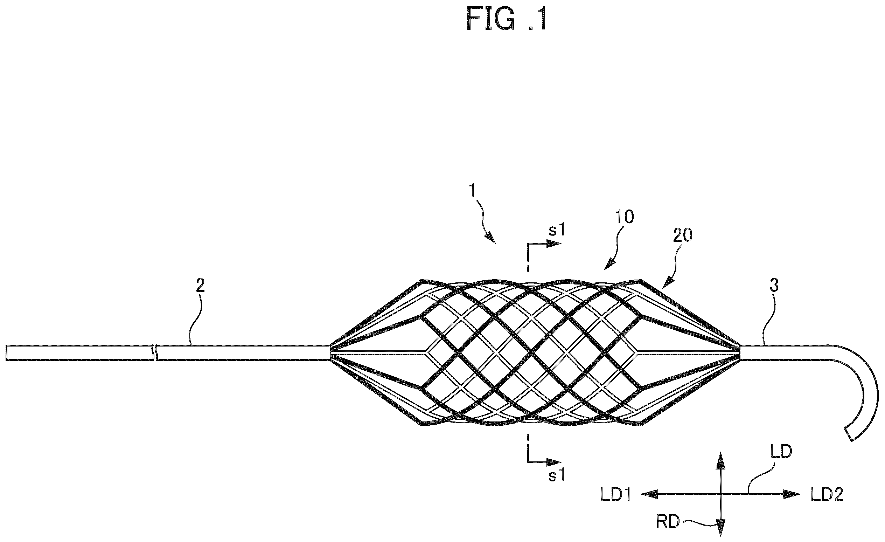

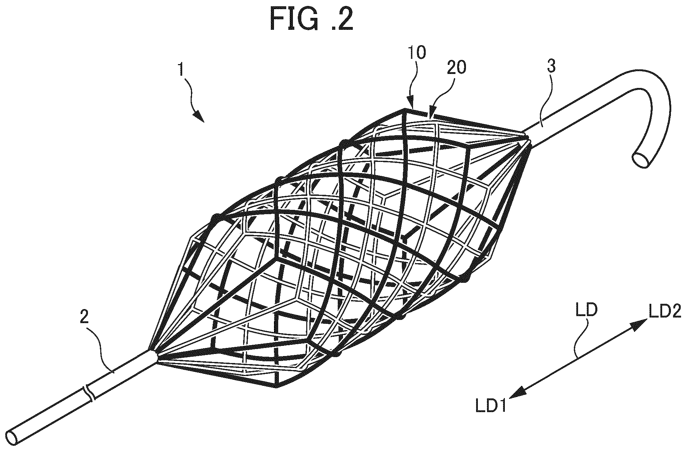

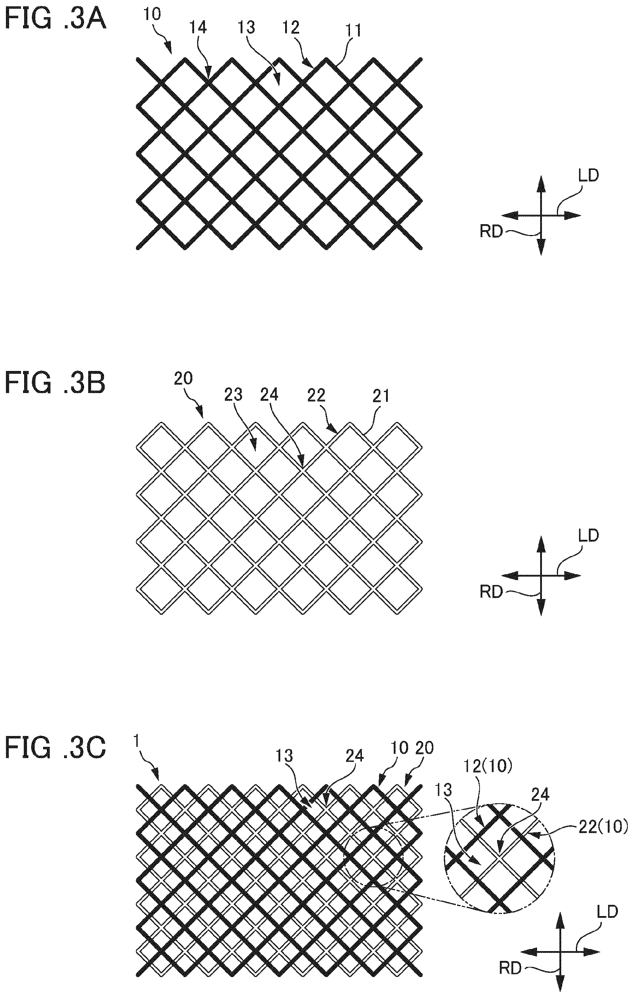

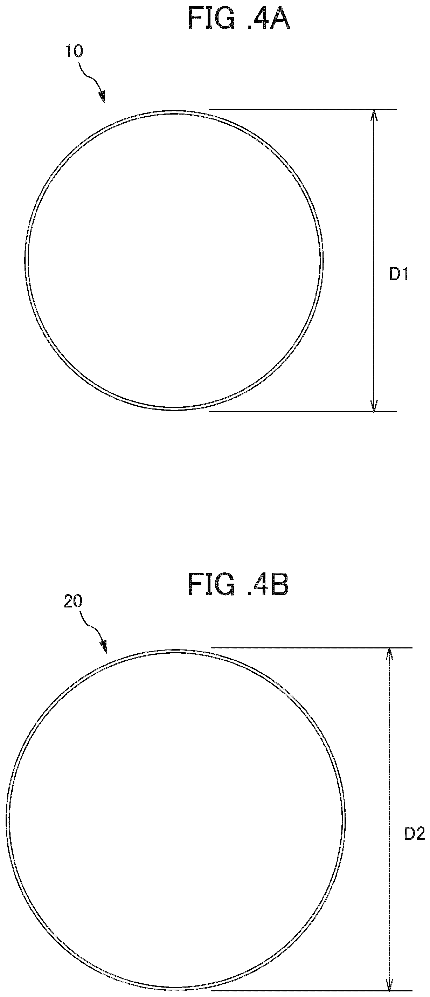

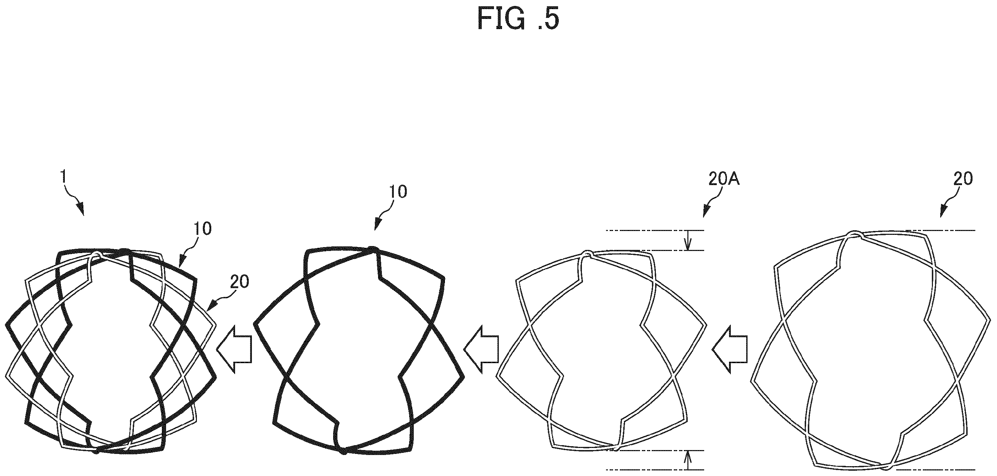

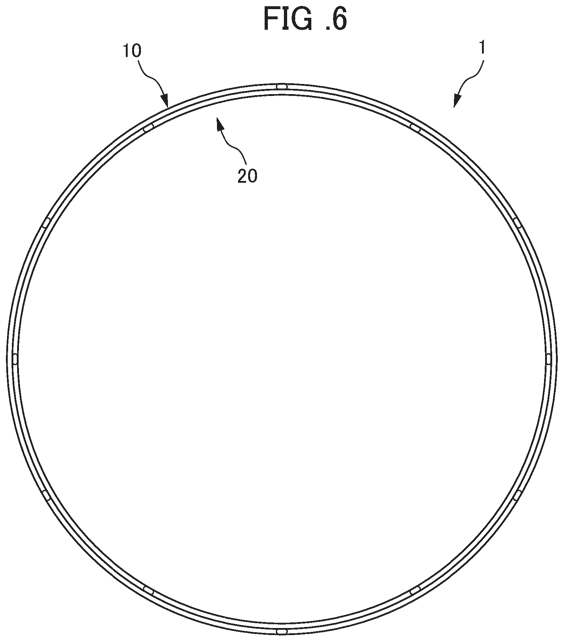

FIG. 1 is a schematic side view of a stent 1 of a first embodiment. FIG. 2 is a schematic perspective view of the stent 1 shown in FIG. 1. FIG. 3A is a development view showing a state in which part of a first stent body 10 of the first embodiment is virtually opened in a planar shape. FIG. 3B is a development view showing a state in which part of a second stent body 20 of the first embodiment is virtually opened in a planar shape. FIG. 3C is a development view showing a state in which part of the stent 1 of the first embodiment is virtually opened in a planar shape. FIG. 4A is a view for describing the outer diameter D1 of the simple first stent body 10. FIG. 4B is a view for describing the outer diameter D2 of the simple second stent body 20. FIG. 5 is a view for describing steps of inserting the second stent body 20 into the first stent body 10. FIG. 6 is a sectional view along an s1-s1 line of FIG. 1.

For the sake of easy distinguishing of the first stent body 10 and the second stent body 20 in the drawings showing the first embodiment and other embodiments, a strut of the first stent body 10 is indicated by black, and a strut of the second stent body 20 is indicated by white. Moreover, in the present specification etc., the "cell" indicates a portion surrounded by a wire-like material forming a mesh pattern. The "cell" includes not only a form in which a shape and a size are the same over the stent body, but also a form in which a shape and a size are different. The "strut" indicates an elongated band-shaped portion made of the wire-like material. In the present specification etc., a cell opening will also be referred to as a "hole", and a portion where struts of adjacent cells are connected to or overlap with each other will also be referred to as an "intersection". Of the intersection, a point at which struts cross each other will also be referred to as an "intersecting point". The intersection may have a certain region (area). The intersection may include a plurality of intersecting points.

The stent 1 of the first embodiment is used, for example, as follows: the stent 1 is sheathed (housed) in (inserted into) a catheter (not shown) and is pushed out of the catheter and is deployed in a vascular lumen, and in this manner, expands a narrowed or occluded blood vessel. As shown in FIGS. 1 and 2, the stent 1 is substantially in a cylindrical shape in a diameter-expanded state. Although not shown in the figure, the stent 1 is in an elongated cylindrical shape in a diameter-narrowed state. A push wire 2 is connected to a proximal side LD1 end portion of the stent 1, and a distal side LD2 end portion of the stent 1 is connected to a distal end shaft 3. Examples of a method for connecting the proximal side end portion of the stent 1 and the push wire 2 to each other may include welding, UV bonding, and silver solder infiltration, but is not particularly limited as long as the connection method is used for general medical equipment. Note that the form of connection between the proximal side end portion of the stent 1 and the push wire 2 will be described later.

The push wire 2 is a member to be operated by the practitioner to move the stent 1. The practitioner pushes or pulls the push wire 2 via an operator (not shown) coupled to the proximal side LD1 of the push wire 2, thereby moving the stent 1 back and forth in the catheter or the blood vessel. The practitioner moves the push wire 2 back and forth, thereby temporarily implanting the stent 1 in a lesion area or recovering the stent 1 from a lesion area. The distal end shaft 3 is a member serving as a mark for checking the distal side LD2 position of the stent 1 on an X-ray transparent image, and for example, the entirety or part of the distal end shaft 3 is made of a material having a high radiopacity. The material having the high radiopacity indicates a material through which no radiation such as an X-ray penetrates or which has a low radiant transmittance. Note that the distal end shaft 3 may be made, for example, of the same material as that of the push wire 2.

The stent 1 includes the first stent body 10 and the second stent body 20. The first stent body 10 is a substantially cylindrical structure arranged outside the stent 1. The second stent body 20 is a substantially cylindrical structure arranged inside the first stent body 10. The stent 1 is a stent having such a double-layer structure in which the second stent body 20 is inserted into the first stent body 10. In a state in which the second stent body 20 is inserted into the first stent body 10, the first stent body 10 and the second stent body 20 are not coupled to each other in the radial direction. Specifically, the first stent body 10 and the second stent body 20 are coupled to each other via the push wire 2 or the distal end shaft 3, but are not coupled to each other between the push wire 2 and the distal end shaft 3. Thus, the stent 1 is configured such that the first stent body 10 and the second stent body 20 are independently deformable on the same layer.

As described later, the stent 1 of the first embodiment is produced in such a manner that the second stent body 20 having a greater outer diameter than that of the first stent body 10 is inserted, in a diameter-narrowed state, into the first stent body 10. Thus, in the stent 1, the inserted second stent body 20 constantly presses the first stent body 10 outward in the radial direction RD. With this configuration, the stent 1 is configured so that the first stent body 10 and the second stent body 20 can be more closely in contact with each other while the state in which the first stent body 10 and the second stent body 20 are independently deformable on the same layer is maintained.

As shown in FIG. 1, on the proximal side LD1 of the stent 1, end portions of the first stent body 10 and the second stent body 20 are gradually narrowed toward the push wire 2, and are connected to the push wire 2. Similarly, on the distal side LD2 of the stent 1, end portions of the first stent body 10 and the second stent body 20 are gradually narrowed toward the distal end shaft 3, and are connected to the distal end shaft 3.

As shown in FIG. 3A, the first stent body 10 is configured such that a plurality of outer cells (first cells) 12 including struts 11 arranged in a frame shape spreads in the radial direction (circumferential direction) .RD. In the first stent body 10, the plurality of outer cells 12 spread in the radial direction RD is continuously arranged in the axial direction LD. That is, the first stent body 10 has such a mesh pattern that the plurality of outer cells 12 including the struts 11 arranged in the frame shape spreads in the radial direction RD and is continuous in the axial direction LD. A hole 13 is formed in the outer cell 12. Adjacent ones of the outer cells 12 are connected to each other at an intersecting point 14.

As shown in FIG. 3B, the second stent body 20 is configured such that a plurality of inner cells (second cells) 22 including struts 21 arranged in a frame shape spreads in the radial direction (circumferential direction) RD. In the second stent body 20, the plurality of inner cells 22 spread in the radial direction RD is continuously arranged in the axial direction LD. That is, the second stent body 20 has such a mesh pattern that the plurality of inner cells 22 including the struts 21 arranged in the frame shape spreads in the radial direction RD and is continuous in the axial direction LD. A hole 23 is formed in the inner cell 22. Adjacent ones of the inner cells 22 are connected to each other at an intersecting point 24.

As shown in FIGS. 3A and 3B, in the stent 1 of the first embodiment, the outer cell 12 forming the first stent body 10 and the inner cell 22 forming the second stent body 20 have the same size, shape, and arrangement, as one example. That is, in the first embodiment, the mesh pattern of the first stent body 10 shown in FIG. 3A and the mesh pattern of the second stent body 20 shown in FIG. 3B are substantially the same pattern. Note that the mesh pattern of the first stent body 10 and the mesh pattern of the second stent body 20 may be different from each other.

As shown in FIG. 3C, in the stent 1, the first stent body 10 and the second stent body 20 overlap with each other such that the intersecting point 24 between the inner cells 22 (of the second stent body 20) is arranged in the hole 13 of the outer cell 12 (of the first stent body 10). Specifically, in a configuration in which the intersecting point 24 between the inner cells 22 is arranged in the hole 13 of the outer cell 12, the first stent body 10 and the second stent body 20 overlap with each other such that one intersecting point 24 between the inner cells 22 is arranged in one hole 13 of the outer cell 12. The mesh patterns of the stent bodies overlap with each other as described above, and therefore, the density of the mesh pattern is increased over the entire stent. Thus, the surface area of the stent 1 can be increased. In the stent 1 of the first embodiment, the percentage of a non-hole portion per unit surface area in the portion where the first stent body 10 and the second stent body 20 overlap with each other is 5 to 50%.

As shown in FIGS. 4A and 4B, in the first embodiment, a relationship between the outer diameter D1 of the simple first stent body 10 and the outer diameter D2 of the simple second stent body 20 is set to D1 < D2. Thus, according to the steps indicated by arrows in FIG. 5, the second stent body 20 having a greater outer diameter than that of the first stent body 10 is narrowed into the form of a second stent body 20A, and the second stent body 20A is inserted into the first stent body 10. In this manner, due to expansive force of the second stent body 20 itself, the stent 1 can be produced with such a double-layer structure in which the second stent body 20 closely contacts the first stent body 10 from the inside thereof. Note that for the sake of easy understanding, FIG. 5 shows only an annular circumferential cell line of each stent body.

In the stent 1 produced as described above, the first stent body 10 and the second stent body 20 closely contact each other with no clearance therebetween in the radial direction RD due to the above-described expansive force of the second stent body 20 itself, as shown in FIG. 6. Thus, in the axial direction LD (see FIG. 1) of the stent 1, it is less likely that the positions of the first stent body 10 and the second stent body 20 are relatively misaligned from each other.

In the first embodiment, the second stent body 20 itself inserted in the narrowed state into the first stent body 10 serves as a self-expanding body (elastic body). Thus, the second stent body 20 constantly presses the first stent body 10 outward in the radial direction RD. Consequently, even if the first stent body 10 and the second stent body 20 are not coupled to each other in the radial direction, the first stent body 10 and the second stent body 20 can more closely contact each other. Moreover, since the first stent body 10 and the second stent body 20 are not coupled to each other in the radial direction in the stent 1, the state in which the first stent body 10 and the second stent body 20 are independently deformable on the same layer can be maintained. Further, the stent 1 having the double-layer structure has the total expansive force of the expansive force of the first stent body 10 outside and the expansive force of the second stent body 20 inside. Thus, even if the stent 1 has the same surface area as that of a stent having a single-layer structure, the stent 1 can have a greater expansive force.

As a material forming the stent 1 (the first stent body 10, the second stent body 20), a material itself having a high stiffness and a high biological compatibility is preferred. Examples of such a material include titanium, nickel, stainless steel, platinum, gold, silver, copper, iron, chromium, cobalt, aluminum, molybdenum, manganese, tantalum, tungsten, niobium, magnesium, calcium, and alloy containing these materials. Particularly, the stent 1 is preferably made of a material having superelastic properties, such as nickel titanium (Ni-Ti) alloy. The mesh patterns of the first stent body 10 and the second stent body 20 may be produced, for example, in such a manner that substantially cylindrical tubes made of the above-described material are machined with laser.

As the material of the stent 1, synthetic resin materials such as polyolefin including PE and PP, polyamide, polyvinyl chloride, polyphenylene sulfide, polycarbonate, polyether, and polymethylmethacrylate may also be used. Further, biodegradable resins (biodegradable polymers) such as polylactate (PLA), polyhydroxybutyrate (PHB), polyglycolic acid (PGA), and poly(ε-caprolactone) may also be used. Of these materials, titanium, nickel, stainless steel, platinum, gold, silver, copper, magnesium, or alloy containing these materials are preferred. Examples of such alloy include Ni-Ti alloy, Cu-Mn alloy, Cu-Cd alloy, Co-Cr alloy, Cu-Al-Mn alloy, Au-Cd-Ag alloy, Ti-Al-V alloy, and alloy of magnesium and Zr, Y, Ti, Ta, Nd, Nb, Zn, Ca, Al, Li, Mn, etc. In addition to the materials described above, non-biodegradable resins may be used as the material of the stent 1. As described above, any material may be used to form the stent 1 as long as such a material has a biological compatibility.

The stent 1 may contain a medical agent. The stent 1 containing the medical agent as described herein indicates that the stent 1 releasably carries the medical agent so as to dissolve out the medical agent. Although the medical agent is not limited, a physiologically active substance may be used, for example. Examples of the physiologically active substance include a medical agent for inhibiting intima thickening, a carcinostatic, an immunosuppressant, an antibiotic, an antirheumatic, an antithrombotic, an HMG-CoA reductase inhibitor, an ACE inhibitor, a calcium channel blocker, an antilipemic, an anti-inflammatory, an integrin inhibitor, an antiallergic, an antioxidant, a GPIIbIIIa antagonist, retinoid, flavonoid, carotenoid, a lipid improver, a DNA synthesis inhibitor, a tyrosine kinase inhibitor, an antiplatelet, a vascular smooth muscle growth inhibitor, an anti-inflammatory agent, and interferon, and these medical agents may be used in combination.

For example, in a case where the first stent body 10 and the second stent body 20 are formed using superelastic alloy tubes, tubes having diameters of about 2 to 3 mm are machined with laser, and in this manner, mesh patterns are formed. Thereafter, the mesh patterns are stretched in the radial direction, and in this manner, can be expanded to desired diameters. As described above, the second stent body 20 is inserted into the first stent body 10, and in this manner, the stent 1 having the double-layer structure can be produced. The stent 1 having the double-layer structure is narrowed in the radial direction from the state shown in FIG. 1, and is sheathed in the catheter (not shown). When the stent 1 sheathed in the catheter is pushed out, the shape shown in FIG. 1 is recovered. The stent 1 is made of, e.g., an elastic material such as superelastic alloy or shape-memory alloy so that the above-described shape recovery function can be obtained. Note that production of the stent 1 is not limited to laser machining, and for example, the stent 1 may be produced by other methods such as cutting.

According to the stent 1 of the above-described first embodiment, the following advantageous effects are obtained, for example. The stent 1 of the first embodiment has the double-layer structure of the first stent body 10 and the second stent body 20, and the first stent body 10 and the second stent body 20 overlap with each other such that the intersecting point 24 between the inner cells 22 of the second stent body 20 is arranged in the hole 13 of the outer cell 12 of the first stent body 10 (see FIG. 3C). The mesh patterns of the stent bodies overlap with each other as described above so that the density of the mesh pattern can be increased over the entire stent. Thus, the surface area of the stent 1 can be increased. Consequently, according to the stent 1 of the first embodiment, the narrowed blood vessel can be more uniformly expanded.

The stent 1 of the first embodiment is configured such that in the state in which the second stent body 20 is inserted into the first stent body 10, the first stent body 10 and the second stent body 20 are not coupled to each other in the radial direction. According to the present configuration, the first stent body 10 and the second stent body 20 are independently deformable on the same layer, and a contact state interfering with deformation of these bodies is less likely to occur. Thus, the flexibility of the entire stent can be more enhanced. As described above, even if the stent 1 of the first embodiment has a great surface area, an excessive increase in bending stiffness can be prevented, and therefore, excellent shape followability (followability of shape) to a vascular structure can be exhibited.

Moreover, as described above, in the stent 1 of the first embodiment, the first stent body 10 and the second stent body 20 are independently deformable on the same layer, and therefore, the stent 1 can be narrowed without interference among the struts of each layer. Since the stent 1 of the first embodiment has excellent diameter reducibility (reducibility of diameter), the stent 1 can be easily sheathed even in a thin catheter as compared to a stent having a mesh pattern with a single-layer structure and a great surface area.

Thus, the stent 1 of the first embodiment has a great surface area, and has excellent shape followability to a vascular structure and excellent diameter reducibility. Note that in a case where a wire-like material is braided to form a double-layer structure, the wire-like material also extends among layers, and for this reason, the braided layers are not independently deformable on the same layer. For this reason, even if the surface area of the stent is increased by the braided double-layer structure, it is difficult to obtain shape followability and diameter reducibility as in the stent 1 of the first embodiment.

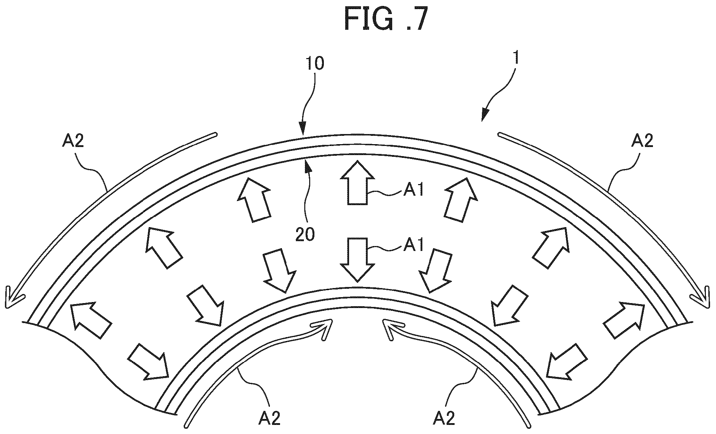

An effect in a case where the stent 1 of the first embodiment is temporarily implanted in a bent blood vessel will be described herein. FIG. 7 is a view for describing an internal state in a case where the stent 1 is bent. FIG. 7 schematically shows the internal state in a case where the stent 1 is temporarily implanted in the bent blood vessel. In FIG. 7, arrows A1 inside the stent 1 indicate the direction of action of the self-expanding force (pressure) of the second stent body 20. As shown in FIG. 7, in the stent 1, the second stent body 20 constantly presses, due to the self-expanding force thereof, the first stent body 10 outward. Thus, in a blood vessel having a small bending radius, a phenomenon so-called "kink" is less likely to occur in the stent 1. The kink indicates that the section of the stent is deformed substantially to an oval shape. Moreover, in the bent blood vessel, a force of buckling the stent 1 is applied as indicated by arrows A2 in FIG. 7. This force is considerable particularly inside the bent portion, and the self-expanding force of the second stent body 20 indicated by the arrows A1 acts so as to face the force indicated by the arrows A2. Thus, even if the bending radius of the stent 1 decreases in the bent blood vessel, the stent 1 is less likely to be bent or kink.

The stent 1 of the above-described first embodiment is sheathed in the catheter, and is deployed in a lesion area in a vascular lumen. In this manner, the vascular lumen can be expanded, and the patency of the lesion area can be ensured. The stent 1 is recovered, without implanted for a long period of time, after a lapse of a predetermined period so that occurrence of restenosis or reocclusion in the blood vessel after implantation of the stent or occurrence of a defect leading to a complication such as a thrombosis can be reduced. Moreover, the stent 1 of the first embodiment has excellent shape followability, and therefore, the blood vessel is less likely to be in a linear shape as compared to a case where a vascular lumen is expanded by a balloon. Thus, the stent 1 of the first embodiment is less likely to cause a hemorrhagic complication due to, e.g., blood vessel damage or rupture or infarction of a penetrating branch of a peripheral blood vessel. Note that the stent 1 of the first embodiment is not limited to use against coarctation in a vascular lumen, and for example, may also be used against coarctation in an organ of the gastrointestinal system, such as the esophagus or the large intestine. That is, the stent 1 of the first embodiment can be used generally for body tissues with lumen structures.

The stent 1 of the first embodiment can also be used for treatment of a cerebrovascular spasm that a cerebral blood vessel is narrowed due to a spasm. As one of a treatment method for the cerebrovascular spasm, a blood vessel is expanded by a balloon. However, in the treatment using the balloon, there is a probability that, e.g., vascular occlusion or blood vessel damage occurs. On the other hand, since the stent 1 of the first embodiment has excellent shape followability as described above, a blood vessel is less likely to be in a linear shape as compared to a case where a vascular lumen is expanded by a balloon. Thus, it is assumed that the stent 1 of the first embodiment is less likely to cause a hemorrhagic complication due to, e.g., blood vessel damage or rupture or infarction of a penetrating branch of a peripheral blood vessel even in a case where the stent 1 is used for treatment of the cerebrovascular spasm. Note that stents of other embodiments to be described later also produce advantageous effects similar to those of the stent 1 of the first embodiment.

### (Second Embodiment)

Next, a stent 1A of a second embodiment will be described. The stent 1A of the second embodiment is different from that of the first embodiment in the cell shapes of first and second stent bodies. Other configurations of the stent 1A of the second embodiment are the same as those of the first embodiment. Thus, in the second embodiment, the entirety of the stent 1A is not shown in the figure. Moreover, in description below and the drawings, the same reference numerals are used as end numerals (last two digits) of elements having functions similar to those of the first embodiment, and overlapping description thereof will be omitted as necessary.

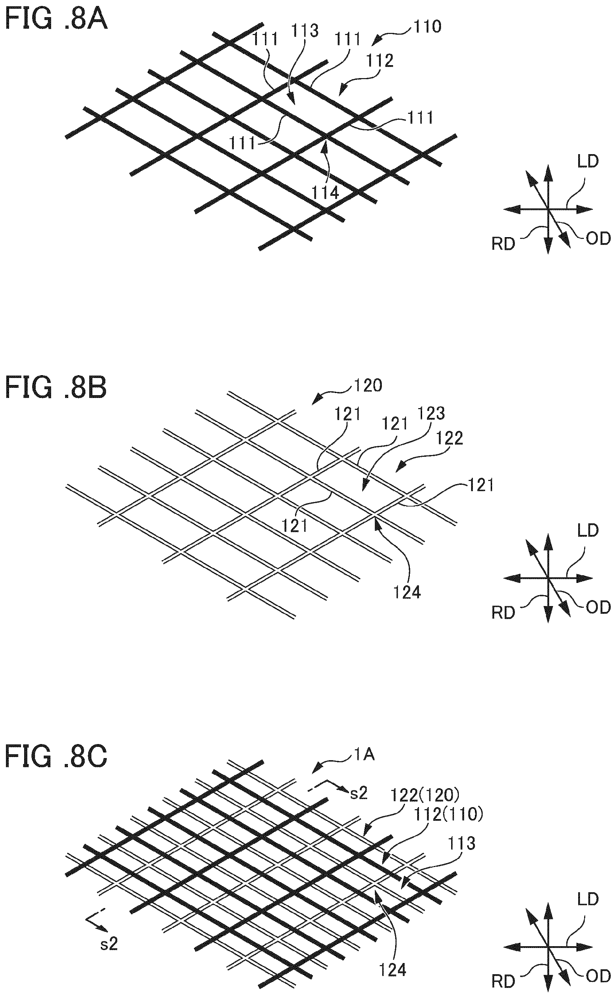

FIG. 8A is a development view showing a state in which a first stent body 110 of the second embodiment is virtually opened in a planar shape. FIG. 8B is a development view showing a state in which a second stent body 120 of the second embodiment is virtually opened in a planar shape. FIG. 8C is a development view showing a state in which the stent 1A of the second embodiment is virtually opened in a planar shape. In the second embodiment, a circumferential direction OD is inclined with respect to a radial direction RD.

The first stent body 110 of the second embodiment is configured, as shown in FIG. 8A, such that a plurality of outer cells (first cells) 112 spreads in the circumferential direction OD. In the first stent body 110, the plurality of outer cells 112 spread in the circumferential direction OD is continuously arranged in an axial direction LD. That is, the first stent body 110 has such a mesh pattern that the plurality of outer cells 112 spreads in the circumferential direction OD and is continuously arranged in the axial direction LD.

The outer cell 112 includes two struts 111 arranged on long sides and two struts 111 arranged on short sides. The outer cell 112 is configured, when opened in a planar shape, such that the long-side struts 111 and the short-side struts 111 are diagonally coupled substantially in the form of a parallelogram. The outer cell 112 has a hole 113. Adjacent ones of the outer cells 112 are connected to each other at an intersecting point 114.

The second stent body 120 of the second embodiment is configured, as shown in FIG. 8B, such that a plurality of inner cells (second cells) 122 spreads in the circumferential direction OD. In the second stent body 120, the plurality of inner cells 122 spread in the circumferential direction OD is continuously arranged in the axial direction LD. That is, the second stent body 120 has such a mesh pattern that the plurality of inner cells 122 spreads in the circumferential direction OD and is continuously arranged in the axial direction LD.

The inner cell 122 includes two struts 121 arranged on long sides and two struts 121 arranged on short sides. The inner cell 122 is configured, when opened in a planar shape, such that the long-side struts 121 and the short-side struts 121 are diagonally coupled substantially in the form of a parallelogram. The inner cell 122 has a hole 123. Adjacent ones of the inner cells 122 are connected to each other at an intersecting point 124.

In the stent 1A of the second embodiment, the plurality of outer cells 112 forming the first stent body 110 and the plurality of inner cells 122 forming the second stent body 120 have the same size, shape, and arrangement, as one example. That is, in the second embodiment, the mesh pattern of the first stent body 110 shown in FIG. 8A and the mesh pattern of the second stent body 120 shown in FIG. 8B are substantially the same pattern. Note that the mesh pattern of the first stent body 110 and the mesh pattern of the second stent body 120 may be different from each other.

As shown in FIG. 8C, in the stent 1A of the second embodiment, the first stent body 110 and the second stent body 120 overlap with each other such that the intersecting point 124 between the inner cells 122 (of the second stent body 120) is arranged in the hole 113 of the outer cell 112 (of the first stent body 110). Specifically, in a configuration in which the intersecting point 124 between the inner cells 122 is arranged in the hole 113 of the outer cell 112, the first stent body 110 and the second stent body 120 overlap with each other such that one intersecting point 124 between the inner cells 122 is arranged in one hole 113 of the outer cell 112. In a case where each stent body is configured, as in the second embodiment, with the cell shape shown in FIGS. 8A and 8B, the mesh patterns of the stent bodies overlap with each other as described above so that the density of the mesh pattern is increased over the entire stent and the surface area of the stent 1A can be more increased.

### (Third Embodiment)

Next, a stent 1B of a third embodiment will be described. The stent 1B of the third embodiment is different from that of the first embodiment in the cell shapes of first and second stent bodies. Other configurations of the stent 1B of the third embodiment are the same as those of the first embodiment. Thus, in the third embodiment, the entirety of the stent 1B is not shown in the figure. Moreover, in description below and the drawings, the same reference numerals are used as end numerals (last two digits) of elements having functions similar to those of the first embodiment, and overlapping description thereof will be omitted as necessary.

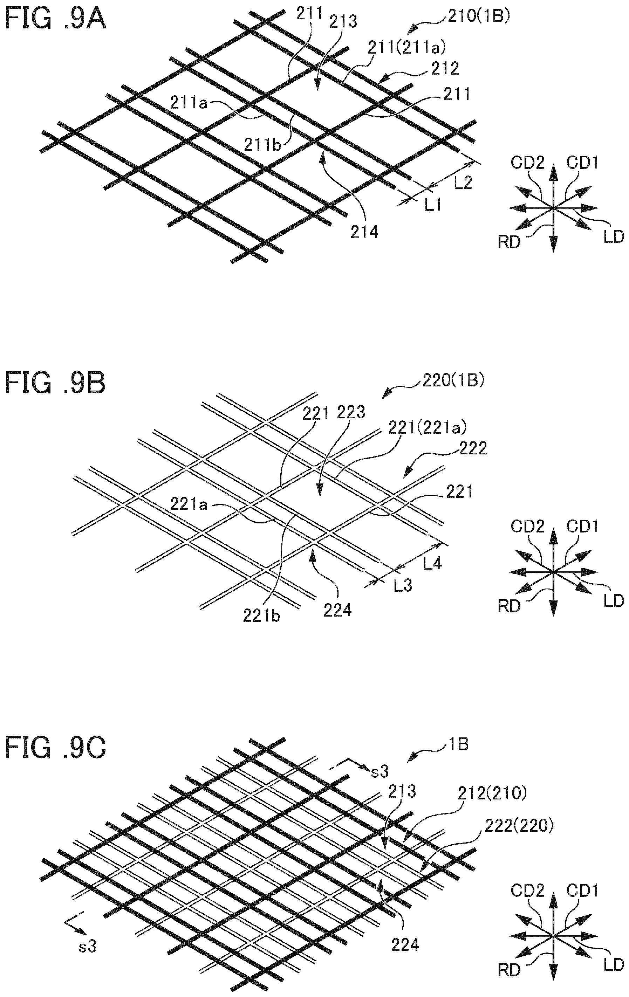

FIG. 9A is a development view showing a state in which a first stent body 210 of the third embodiment is virtually opened in a planar shape. FIG. 9B is a development view showing a state in which a second stent body 220 of the third embodiment is virtually opened in a planar shape. FIG. 9C is a development view showing a state in which the stent 1B of the third embodiment is virtually opened in a planar shape. In the third embodiment, annular directions of cells being connected diagonally to a radial direction (circumferential direction) RD will be referred to as annular directions CD1, CD2.

The first stent body 210 of the third embodiment is configured, as shown in FIG. 9A, such that a plurality of outer cells (first cells) 212 spreads in the radial direction (circumferential direction) RD. In the first stent body 210, the plurality of outer cells 212 spread in the radial direction RD is continuously arranged in an axial direction LD. That is, the first stent body 210 has such a mesh pattern that the plurality of outer cells 212 spreads in the radial direction RD and is continuously arranged in the axial direction LD.

The outer cell 212 includes, in the annular direction CD1, a pair of struts (first struts) 211 (hereinafter also referred to as "211a to 211b") and a strut (first strut) 211 arranged with a clearance (a hole 213) from the pair of struts 211. Moreover, the outer cell 212 includes, in the annular direction CD2, two struts 211 arranged with a clearance (the hole 213) therebetween so as to face each other. The ratio of a clearance L1 between the pair of struts 211a to 211b to the clearance L2 of the hole 213 is about 1:3 to 1:10, for example. The strut 211 arranged apart from the pair of struts 211 in the annular direction CD1 in a certain outer cell 212 is one strut 211a of the pair of struts 211 in another outer cell 212 adjacent to the certain outer cell 212 in the annular direction CD1.

In the outer cell 212, the hole 213 is formed. In each outer cell 212 arranged along the annular direction CD1, the pair of struts 211a to 211b and struts 211 extending along the annular direction CD1 are connected to each other at intersections 214.

The second stent body 220 of the third embodiment is configured, as shown in FIG. 9B, such that a plurality of inner cells (second cells) 222 spreads in the radial direction (circumferential direction) RD. In the second stent body 220, the plurality of inner cells 222 spread in the radial direction RD is continuously arranged in the axial direction LD. That is, the second stent body 220 has such a mesh pattern that the plurality of inner cells 222 spreads in the radial direction RD and is continuously arranged in the axial direction LD.

The inner cell 222 includes, in the annular direction CD1, a pair of struts (second struts) 221 (hereinafter also referred to as "221a to 221b") and one strut (second strut) 221 arranged with a clearance (a hole 223) from the pair of struts 221. Moreover, the inner cell 222 includes, in the annular direction CD2, two struts 221 arranged with a clearance (the hole 223) therebetween so as to face each other. The ratio of a clearance L3 between the pair of struts 221a to 221b to the clearance L4 of the hole 223 is about 1:3 to 1:10, for example. The strut 221 arranged apart from the pair of struts 211 in the annular direction CD1 in a certain inner cell 222 is one strut 221a of the pair of struts 221 in another inner cell 222 adjacent to the certain inner cell 222 in the annular direction CD1.

In the inner cell 222, the hole 223 is formed. In each inner cell 222 arranged along the annular direction CD1, the pair of struts 221a to 221b and struts 221 extending along the annular direction CD1 are connected to each other at intersections 224.

In the stent 1B of the third embodiment, the plurality of outer cells 212 forming the first stent body 210 and the plurality of inner cells 222 forming the second stent body 220 have the same size, shape, and arrangement, as one example. That is, in the third embodiment, the mesh pattern of the first stent body 210 shown in FIG. 9A and the mesh pattern of the second stent body 220 shown in FIG. 9B are substantially the same pattern. Note that the mesh pattern of the first stent body 210 and the mesh pattern of the second stent body 220 may be different from each other.

As shown in FIG. 9C, in the stent 1B, the first stent body 210 and the second stent body 220 overlap with each other such that the intersection 224 between the inner cells 222 (of the second stent body 220) is arranged in the hole 213 of the outer cell 212 (of the first stent body 210). Specifically, in a configuration in which the intersection 224 between the inner cells 222 is arranged in the hole 213 of the outer cell 212, the first stent body 210 and the second stent body 220 overlap with each other such that one intersection 224 between the inner cells 222 is arranged in one hole 213 of the outer cell 212. In a case where each stent body is configured, as in the third embodiment, with the cell shape shown in FIGS. 9A and 9B, the mesh patterns of the stent bodies overlap with each other as described above so that the density of the mesh pattern is increased over the entire stent and the surface area of the stent 1B can be more increased.

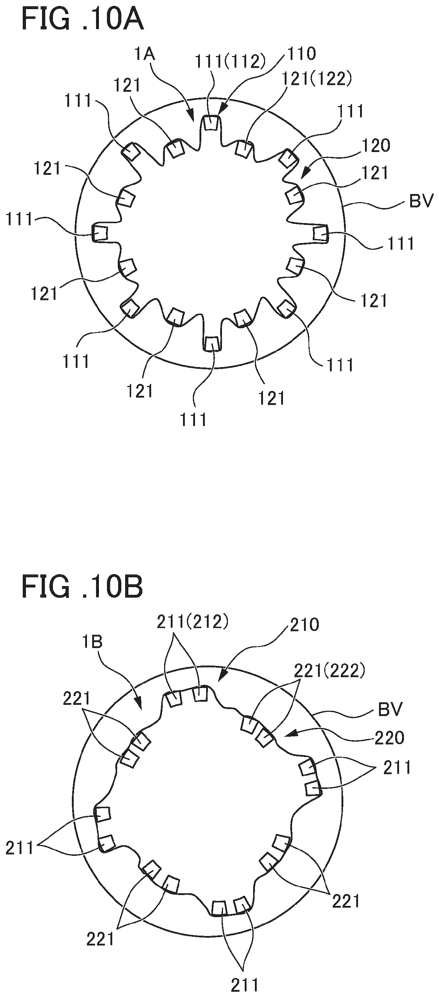

Next, other advantageous effects of the stent 1B of the third embodiment will be described. FIG. 10A is a sectional view in a case where the stent 1A of the above-described second embodiment is expanded in a blood vessel. FIG. 10A virtually shows the section of the stent 1A expanded in the blood vessel, for example, in a case where the stent 1A is diagonally cut along an s2-s2 line of FIG. 8C. FIG. 10B is a sectional view in a case where the stent 1B of the third embodiment is expanded in a blood vessel. FIG. 10B virtually shows the section of the stent 1B expanded in the blood vessel, for example, in a case where the stent 1B is diagonally cut along an s3-s3 line of FIG. 9C.

When the stent 1A of the second embodiment is expanded in a blood vessel BV as shown in FIG. 10A, the struts 111 forming the outer cells 112 of the first stent body 110 and the struts 121 forming the inner cells 122 of the second stent body 120 are alternately arranged in a circumferential direction. Thus, the section of the opening of the stent 1A is in a shape greatly corrugated along the circumferential direction and having large steps. Note that the "circumferential direction" in description of FIGS. 10A and 10B means a circumferential direction when the stent is viewed in the axial direction (center axis direction).

On the other hand, when the stent 1B of the third embodiment is expanded in the blood vessel BV as shown in FIG. 10B, the pairs of struts 211 (211a to 211b: see FIG. 9A) forming the outer cells 212 of the first stent body 210 and the pairs of struts 221 (221a to 221b: see Fig. 9B) forming the inner cells 222 of the second stent body 220 are alternately arranged along the circumferential direction. Thus, the section of the opening of the stent 1B is in a shape less corrugated along the circumferential direction and having smaller steps. As described above, since the stent 1B of the third embodiment is less corrugated in the section of the opening, the stent 1B can be expanded in a shape closer to a circular shape. Thus, according to the stent 1B of the third embodiment, an inner wall of the narrowed blood vessel BV can be more uniformly expanded.

### (Fourth Embodiment)

Next, a stent 1C of a fourth embodiment will be described. The stent 1C of the fourth embodiment is different from that of the third embodiment in the cell shapes of first and second stent bodies. Thus, in description and drawings of the fourth embodiment, the same reference numerals are used as end numerals (last two digits) of elements having functions similar to those of the third embodiment, and overlapping description thereof will be omitted as necessary. In the fourth embodiment, annular directions of cells being connected diagonally to a radial direction (circumferential direction) RD will be referred to as annular directions CD1, CD2.

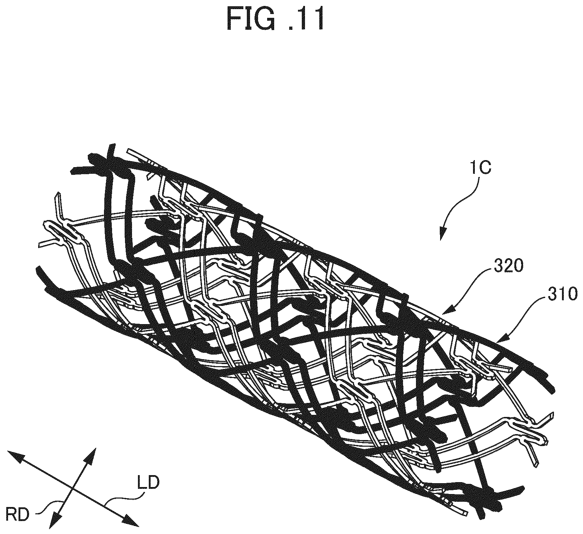

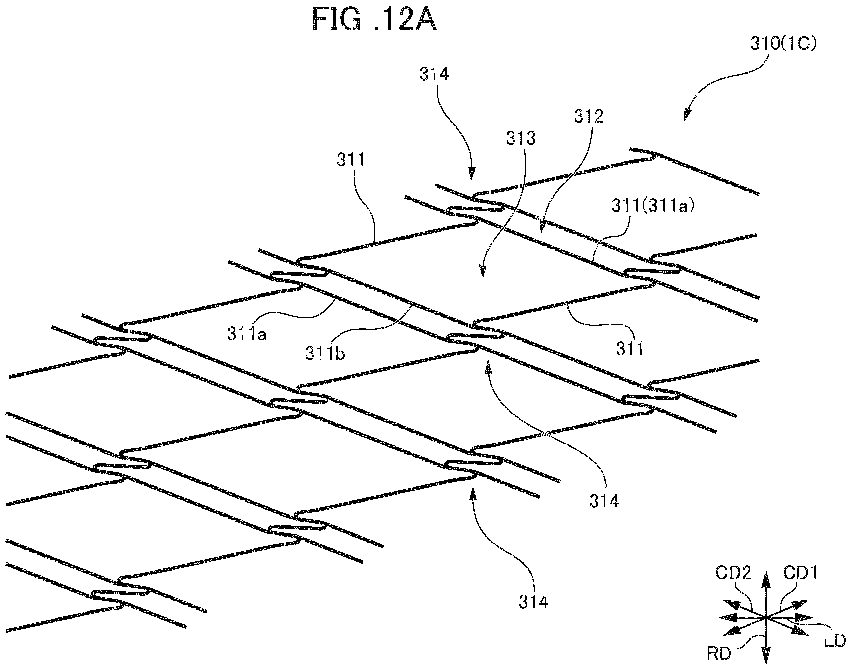

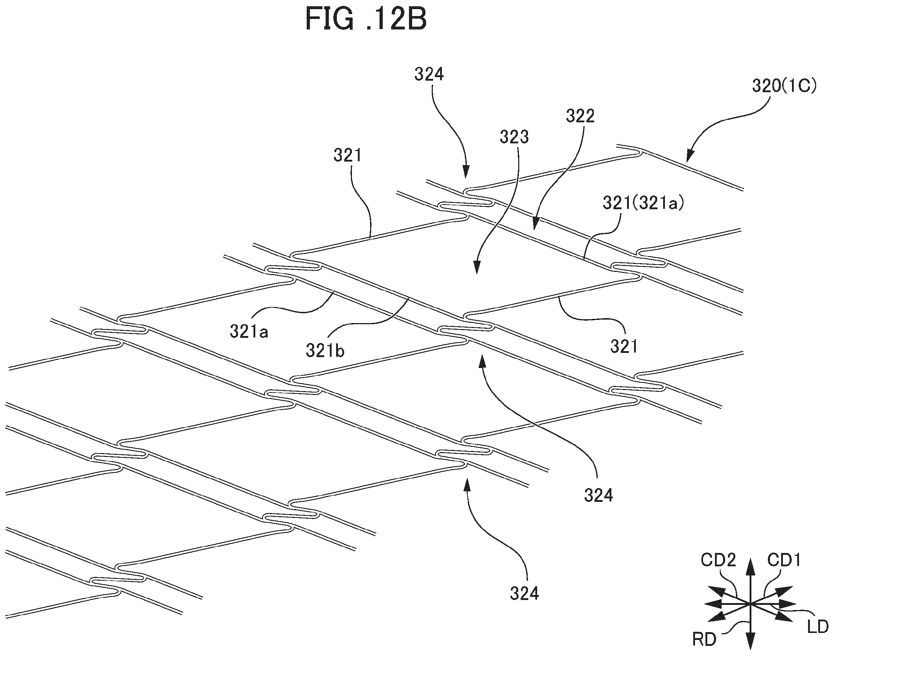

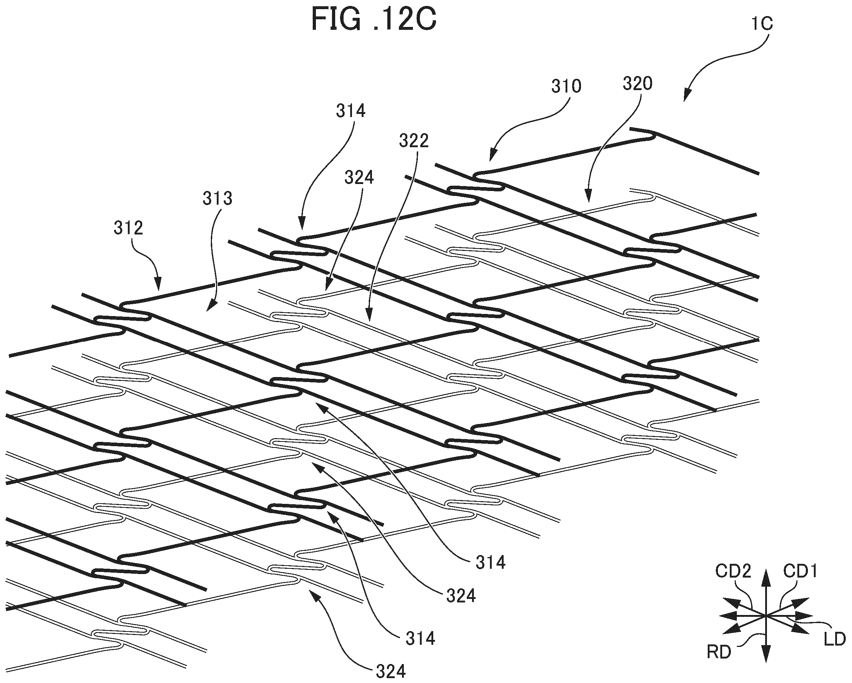

FIG. 11 is a schematic perspective view of the stent 1C of the fourth embodiment. FIG. 12A is a development view showing a state in which a first stent body 310 of the fourth embodiment is virtually opened in a planar shape. FIG. 12B is a development view showing a state in which a second stent body 320 of the fourth embodiment is virtually opened in a planar shape. FIG. 12C is a development view showing a state in which the stent 1C of the fourth embodiment is virtually opened in a planar shape.

As shown in FIG. 11, the stent 1C of the fourth embodiment includes the first stent body 310 and the second stent body 320. The first stent body 310 is a substantially cylindrical structure arranged outside the stent 1C. The second stent body 320 is a substantially cylindrical structure arranged inside the first stent body 310. The stent 1C has such a double-layer structure in which the second stent body 320 is inserted into the first stent body 310. Note that FIG. 11 does not show the push wire 2 and the distal end shaft 3 shown in FIG. 1.

The first stent body 310 is configured, as shown in FIG. 12A, such that a plurality of outer cells (first cells) 312 spreads in the radial direction (circumferential direction) RD. In the first stent body 310, the plurality of outer cells 312 spread in the radial direction RD is continuously arranged in an axial direction LD. That is, the first stent body 310 has such a mesh pattern that the plurality of outer cells 212 spreads in the radial direction RD and is continuously arranged in the axial direction LD.

The outer cell 312 includes, in the annular direction CD1, a pair of struts (first struts) 311 (hereinafter also referred to as "311a to 311b") and one strut (first strut) 311 arranged with a clearance (a hole 313) from the pair of struts 311. Moreover, the outer cell 312 includes, in the annular direction CD2, two struts 311 arranged with a clearance (the hole 313) therebetween so as to face each other. The strut 311 arranged apart from the pair of struts 311 in the annular direction CD1 in a certain outer cell 312 is one strut 311a of the pair of struts 311 in another outer cell 312 adjacent to the certain outer cell 312 in the annular direction CD1.

In the outer cell 312, the hole 313 is formed. In each outer cell 312 arranged along the annular direction CD1, the pair of struts 311a to 311b and struts 311 extending along the annular direction CD1 are connected to each other at substantially S-shaped first intersections 314. The first intersection 314 deforms so as to stretch in the radial direction RD when the expanded stent 1C is bent substantially in a U-shape (see FIG. 7). Thus, the outer cells 312 spread in the radial direction RD can be more flexibly bent. As shown in FIG. 12A, in the first stent body 310, the first intersections 314 are arranged in parallel in the radial direction RD.

The second stent body 320 is configured, as shown in FIG. 12B, such that a plurality of inner cells (second cells) 322 spreads in the radial direction (circumferential direction) RD. In the second stent body 320, the plurality of inner cells 322 spread in the radial direction RD is continuously arranged in the axial direction LD. That is, the second stent body 320 has such a mesh pattern that the plurality of inner cells 322 spreads in the radial direction RD and is continuously arranged in the axial direction LD.

The inner cell 322 includes, in the annular direction CD1, a pair of struts (second struts) 321 (hereinafter also referred to as "321a to 321b") and one strut (second strut) 321 arranged with a clearance (a hole 323) from the pair of struts 321. Moreover, the inner cell 322 includes, in the annular direction CD2, two struts 321 arranged with a clearance (the hole 323) therebetween so as to face each other. The strut 321 arranged apart from the pair of struts 321 in the annular direction CD1 in a certain inner cell 322 is one strut 321a of the pair of struts 321 in another inner cell 322 adjacent to the certain inner cell 322 in the annular direction CD1.

In the inner cell 322, the hole 323 is formed. In each inner cell 322 arranged along the annular direction CD1, the pair of struts 321a to 321b and struts 321 extending along the annular direction CD1 are connected to each other at substantially S-shaped second intersections 324. The second intersection 324 deforms so as to stretch in the radial direction RD when the expanded stent 1C is bent substantially in the U-shape (see FIG. 7). Thus, the inner cells 322 spread in the radial direction RD can be more flexibly bent. As shown in FIG. 12B, in the second stent body 320, the second intersections 324 are arranged in parallel in the radial direction RD.

In the stent 1C of the fourth embodiment, the plurality of outer cells 312 forming the first stent body 310 and the plurality of inner cells 322 forming the second stent body 320 have the same size, shape, and arrangement, as one example. That is, in the fourth embodiment, the mesh pattern of the first stent body 310 shown in FIG. 12A and the mesh pattern of the second stent body 320 shown in FIG. 12B are substantially the same pattern. Note that the mesh pattern of the first stent body 310 and the mesh pattern of the second stent body 320 may be different from each other.

As shown in FIG. 12C, in the stent 1C, the first stent body 310 and the second stent body 320 overlap with each other such that the second intersection 324 between the inner cells 322 (of the second stent body 320) is arranged in the hole 313 of the outer cell 312 (of the first stent body 310). Specifically, in a configuration in which the second intersection 324 between the inner cells 322 is arranged in the hole 313 of the outer cell 312, the first stent body 310 and the second stent body 320 overlap with each other such that one second intersection 324 between the inner cells 322 is arranged in one hole 313 of the outer cell 312. In a case where each stent body is configured, as in the fourth embodiment, with the cell shape shown in FIGS. 12A and 12B, the mesh patterns of the stent bodies overlap with each other as described above so that the density of the mesh pattern is increased over the entire stent and the surface area of the stent 1C can be more increased.

In the stent 1C of the fourth embodiment, the outer cells 312 of the first stent body 310 are connected, in the annular direction CD1, to each other at the substantially S-shaped first intersections 314. Similarly, the inner cells 322 of the second stent body 320 are connected, in the annular direction CD2, to each other at the substantially S-shaped second intersections 324. The first intersections 314 of the first stent body 310 and the second intersections 324 of the second stent body 320 are arranged in parallel in the radial direction RD, as shown in FIG. 12C. According to the present configuration, the outer cells 312 and the inner cells 322 spread in the radial direction RD can be more flexibly bent when the expanded stent 1C is bent substantially in the U-shape (see FIG. 7), and therefore, the shape followability of the stent 1C can be more enhanced.

### (Fifth Embodiment)

Next, a stent 1D of a fifth embodiment will be described. The stent 1D of the fifth embodiment is different from that of the third embodiment in the cell shapes of first and second stent bodies. Thus, in description and drawings of the fifth embodiment, the same reference numerals are used as end numerals (last two digits) of elements having functions similar to those of the third embodiment, and overlapping description thereof will be omitted as necessary. In the fifth embodiment, annular directions of cells being connected diagonally to a radial direction (circumferential direction) RD will be referred to as annular directions CD1, CD2.

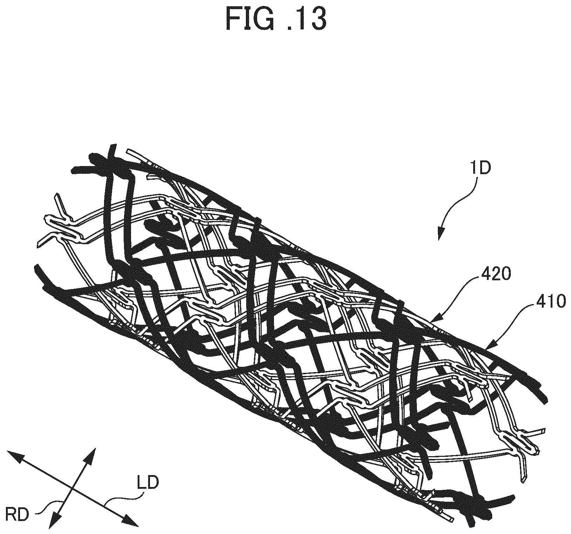

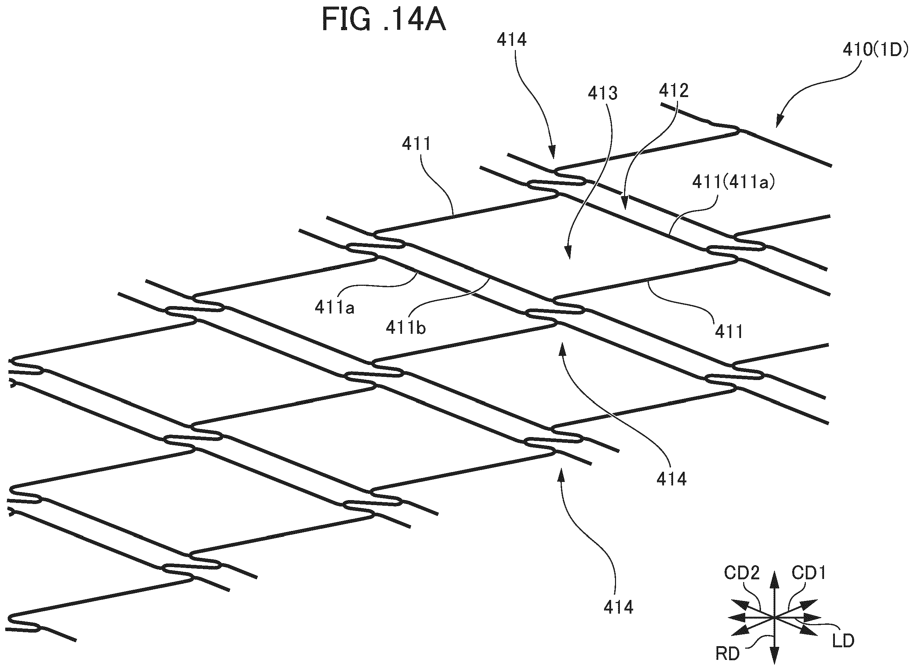

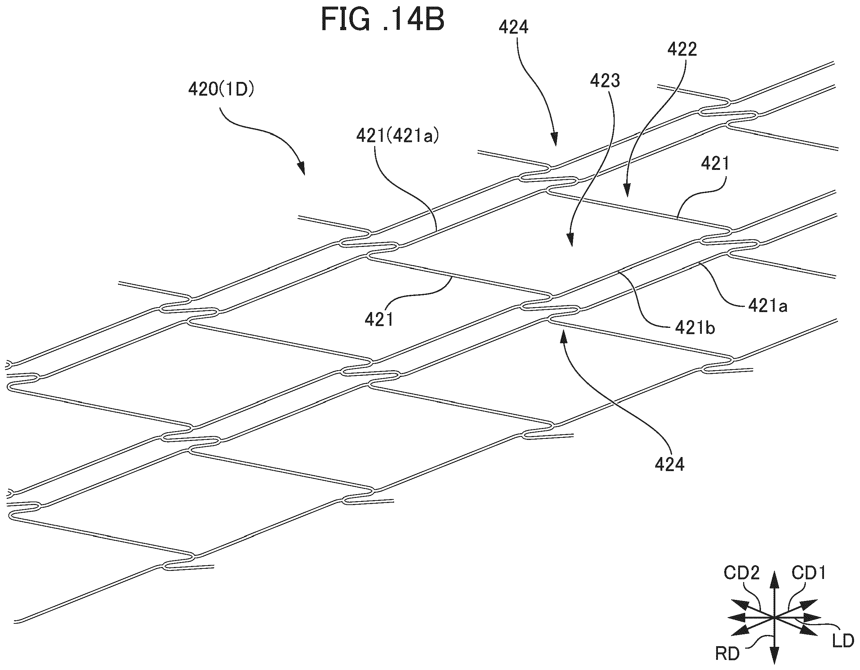

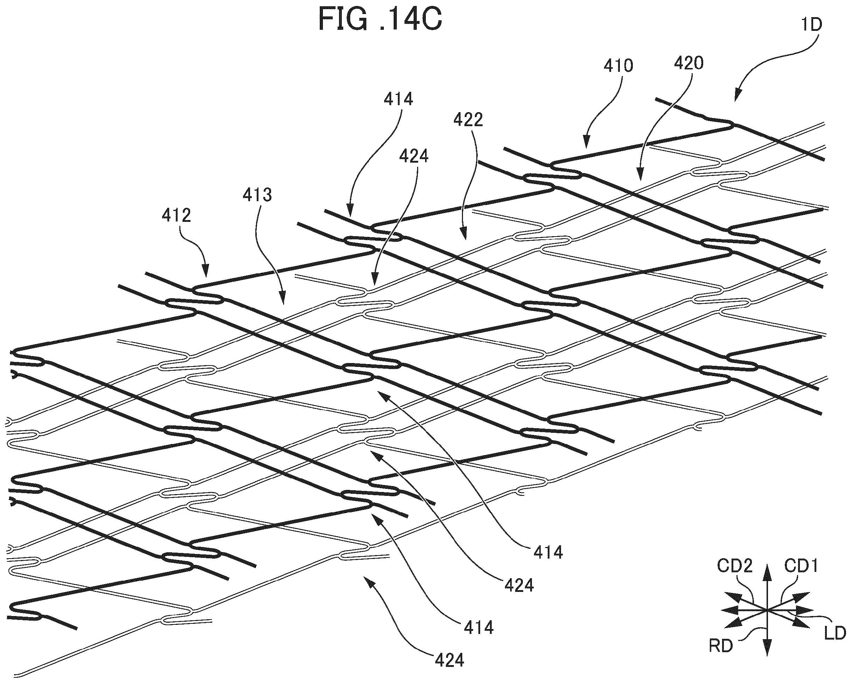

FIG. 13 is a schematic perspective view of the stent 1D of the fifth embodiment. FIG. 14A is a development view showing a state in which a first stent body 410 of the fifth embodiment is virtually opened in a planar shape. FIG. 14B is a development view showing a state in which a second stent body 420 of the fifth embodiment is virtually opened in a planar shape. FIG. 14C is a development view showing a state in which the stent 1D of the fifth embodiment is virtually opened in a planar shape.

As shown in FIG. 13, the stent 1D of the fifth embodiment includes the first stent body 410 and the second stent body 420. The first stent body 410 is a substantially cylindrical structure arranged outside the stent 1D. The second stent body 420 is a substantially cylindrical structure arranged inside the first stent body 410. The stent 1D has such a double-layer structure in which the second stent body 420 is inserted into the first stent body 410. Note that FIG. 13 does not show the push wire 2 and the distal end shaft 3 shown in FIG. 1.

The first stent body 410 of the fifth embodiment is configured, as shown in FIG. 14A, such that a plurality of outer cells (first cells) 412 spreads in the radial direction (circumferential direction) RD. In the first stent body 410, the plurality of outer cells 412 spread in the radial direction RD is continuously arranged in an axial direction LD. That is, the first stent body 410 has such a mesh pattern that the plurality of outer cells 412 spreads in the radial direction RD and is continuously arranged in the axial direction LD.

In the fifth embodiment, the configuration of the first stent body 410 is substantially the same as that of the first stent body 310 of the fourth embodiment, and therefore, detailed description thereof will be omitted. In the first stent body 410 of the fifth embodiment, struts 411, 411a, 411b, the outer cell 412, a hole 413, and a first intersection 414 are equivalent to the struts 311, 311a, 311b, the outer cell 312, the hole 313, and the first intersection 314 of the first stent body 310 of the fourth embodiment. As shown in FIG. 14A, the outer cells 412 of the first stent body 410 are connected to each other at the first intersections 414 in the annular direction CD1.

The second stent body 420 of the fifth embodiment is configured, as shown in FIG. 14B, such that a plurality of inner cells (second cells) 422 spreads in the radial direction (circumferential direction) RD. In the second stent body 420, the plurality of inner cells 422 spread in the radial direction RD is continuously arranged in the axial direction LD. That is, the second stent body 420 has such a mesh pattern that the plurality of inner cells 422 spreads in the radial direction RD and is continuously arranged in the axial direction LD.

The inner cell 422 includes, in the annular direction CD2, a pair of struts (second struts) 421 (hereinafter also referred to as "421a to 421b") and one strut (second strut) 421 arranged with a clearance (a hole 423) from the pair of struts 421. Moreover, the inner cell 422 includes, in the annular direction CD2, two struts 421 arranged with a clearance (the hole 423) therebetween so as to face each other. The strut 421 arranged apart from the pair of struts 421 in the annular direction CD2 in a certain inner cell 422 is one strut 421a of the pair of struts 421 in another inner cell 422 adjacent to the certain inner cell 422 in the annular direction CD2.

In the inner cell 422, the hole 423 is formed. In each inner cell 422 arranged along the annular direction CD2, the pair of struts 421a to 421b and struts 421 extending along the annular direction CD2 are connected to each other at substantially S-shaped second intersections 424. The second intersection 424 deforms so as to stretch in the radial direction RD when the expanded stent 1D is bent substantially in a U-shape (see FIG. 7). Thus, the inner cells 422 spread in the radial direction RD can be more flexibly bent.

As shown in FIG. 14B, the inner cells 422 of the second stent body 420 are connected, in the annular direction CD2, to each other at the second intersections 424. Thus, in the stent 1D of the fifth embodiment, the annular direction CD1 in which the outer cells 412 of the first stent body 410 are connected to each other at the first intersections 414 (see FIG. 14A) and the annular direction CD2 in which the inner cells 422 of the second stent body 420 are connected to each other at the second intersections 424 are symmetrical with respect to a line along the radial direction RD.

As shown in FIG. 14C, in the stent 1D, the first stent body 410 and the second stent body 420 overlap with each other such that the second intersection 424 between the inner cells 422 (of the second stent body 420) is arranged in the hole 413 of the outer cell 412 (of the first stent body 410). Specifically, in a configuration in which the second intersection 424 between the inner cells 422 is arranged in the hole 413 of the outer cell 412, the first stent body 410 and the second stent body 420 overlap with each other such that one second intersection 424 between the inner cells 422 is arranged in one hole 413 of the outer cell 412. Moreover, in the stent 1D, the first intersections 414 of the outer cells 412 and the second intersections 424 of the inner cells 422 are arranged in parallel in the radial direction RD, and are arranged alternately. In a case where each stent body is configured with the cell shape shown in FIGS. 14A and 14B, the mesh patterns of the stent bodies overlap with each other as described above so that the density of the mesh pattern is increased over the entire stent and the surface area of the stent 1D can be more increased.

Further, in the stent 1D of the fifth embodiment, the outer cells 412 of the first stent body 410 are connected, in the annular direction CD1, to each other at the substantially S-shaped first intersections 414. On the other hand, the inner cells 422 of the second stent body 420 are connected, in the annular direction CD2, to each other at the substantially S-shaped second intersections 424. The first intersections 414 of the first stent body 410 and the second intersections 424 of the second stent body 420 are arranged in parallel in the radial direction RD, as shown in FIG. 14C. According to the present configuration, the outer cells 412 and the inner cells 422 spread in the radial direction RD can be more flexibly bent when the expanded stent 1D is bent substantially in the U-shape (see FIG. 7), and therefore, the shape followability of the stent 1D can be more enhanced.

### (Sixth Embodiment)

Next, a stent 1E of a sixth embodiment will be described. The stent 1E of the sixth embodiment is different from that of the third embodiment in the cell shapes of first and second stent bodies. Thus, in description and drawings of the sixth embodiment, the same reference numerals are used as end numerals (last two digits) of elements having functions similar to those of the third embodiment, and overlapping description thereof will be omitted as necessary. In the sixth embodiment, annular directions of cells being connected diagonally to a radial direction (circumferential direction) RD will be referred to as annular directions CD1, CD2.

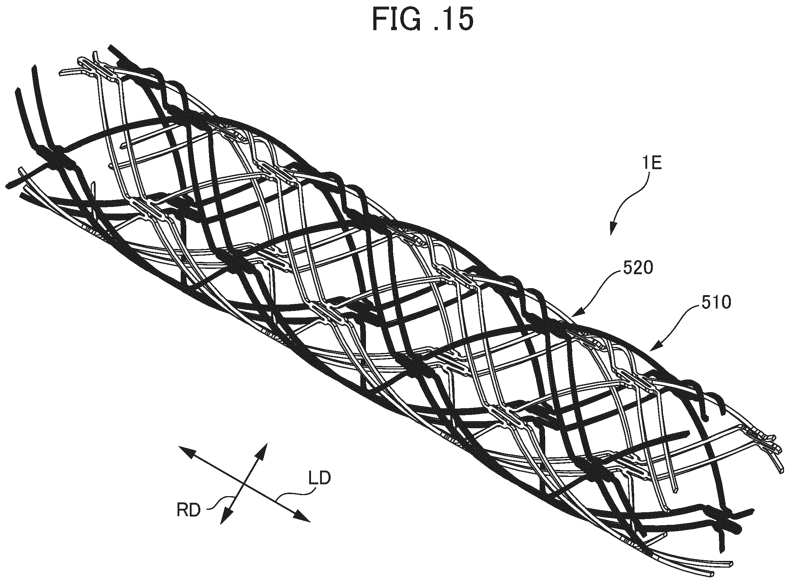

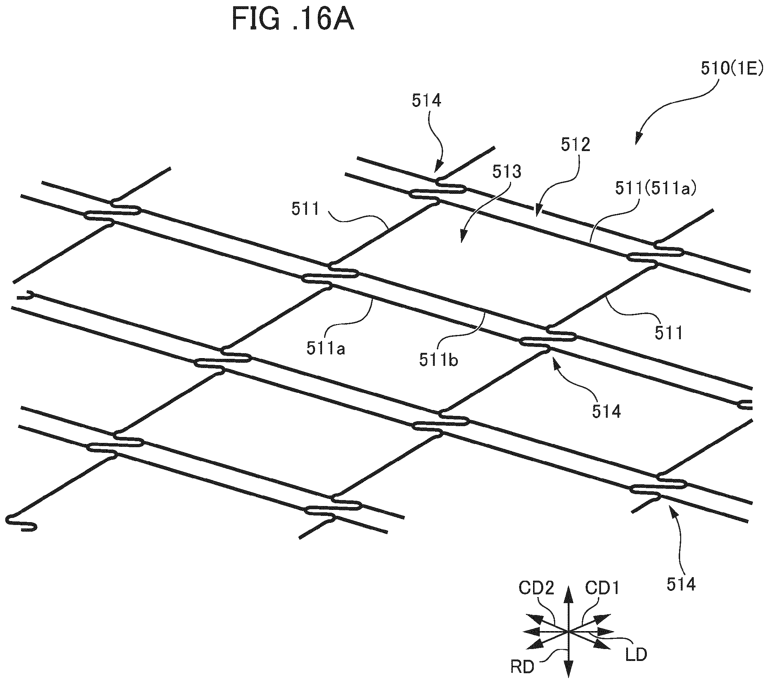

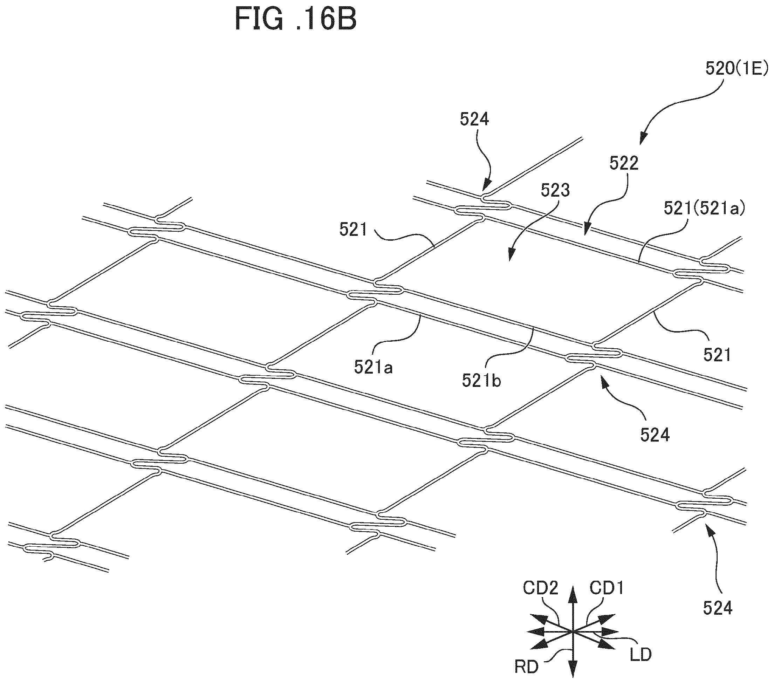

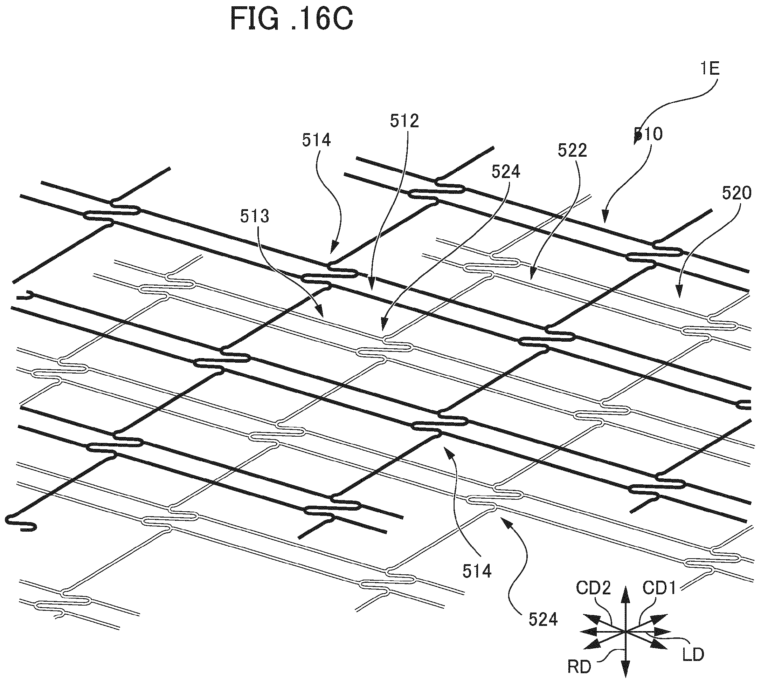

FIG. 15 is a schematic perspective view of the stent 1E of the sixth embodiment. FIG. 16A is a development view showing a state in which a first stent body 510 of the sixth embodiment is virtually opened in a planar shape. FIG. 16B is a development view showing a state in which a second stent body 520 of the sixth embodiment is virtually opened in a planar shape. FIG. 16C is a development view showing a state in which the stent 1E of the sixth embodiment is virtually opened in a planar shape. In the sixth embodiment, a circumferential direction OD is inclined with respect to the radial direction RD.

As shown in FIG. 15, the stent 1E of the sixth embodiment includes the first stent body 510 and the second stent body 520. The first stent body 510 is a substantially cylindrical structure arranged outside the stent 1E. The second stent body 520 is a substantially cylindrical structure arranged inside the first stent body 510. The stent 1E of the sixth embodiment has such a double-layer structure in which the second stent body 520 is inserted into the first stent body 510. Note that FIG. 15 does not show the push wire 2 and the distal end shaft 3 shown in FIG. **1****.**

The first stent body 510 is configured, as shown in FIG. 16A, such that a plurality of outer cells (first cells) 512 spreads in the circumferential direction OD. In the first stent body 510, the plurality of outer cells 512 spread in the circumferential direction OD is continuously arranged in an axial direction LD. That is, the first stent body 510 has such a mesh pattern that the plurality of outer cells 512 spreads in the circumferential direction OD and is continuously arranged in the axial direction LD.

The outer cell 512 includes, in the annular direction CD1, a pair of struts (first struts) 511 (hereinafter also referred to as "511a to 511b") and one strut (first strut) 511 arranged with a clearance (a hole 513) from the pair of struts 511. Moreover, the outer cell 512 includes, in the annular direction CD2, two struts 511 arranged with a clearance (the hole 513) therebetween so as to face each other. The strut 511 arranged apart from the pair of struts 511 in the annular direction CD1 in a certain outer cell 512 is one strut 511a of the pair of struts 511 in another outer cell 512 adjacent to the certain outer cell 512 in the annular direction CD1.

The pair of struts 511a to 511b and one strut 511 arranged in the annular direction CD1 form the long sides of the outer cell 512. Two struts 511 arranged in the annular direction CD2 form the short sides of the outer cell 512. The outer cell 512 is configured, when opened in a planar shape, such that the long-side struts 511a to 511b, 511 and the short-side struts 511 are diagonally coupled substantially in the form of a parallelogram.

In the outer cell 512, the hole 513 is formed. In each outer cell 512 arranged along the annular direction CD1, the pair of struts 511a to 511b and the struts 511 extending along the annular direction CD1 are connected at substantially S-shaped first intersections 514. The first intersection 514 deforms so as to stretch in the radial direction RD when the expanded stent 1E is bent substantially in a U-shape (see FIG. 7). Thus, the outer cells 512 spread in the radial direction RD can be more flexibly bent. As shown in FIG. 16A, in the first stent body 510, the first intersections 514 are arranged in parallel in the circumferential direction OD.

The second stent body 520 is configured, as shown in FIG. 16B, such that a plurality of inner cells (second cells) 522 spreads in the circumferential direction OD. In the second stent body 520, the plurality of inner cells 522 spread in the circumferential direction OD is continuously arranged in the axial direction LD. That is, the second stent body 520 has such a mesh pattern that the plurality of inner cells 522 spreads in the circumferential direction OD and is continuously arranged in the axial direction LD.

The inner cell 522 includes, in the annular direction CD1, a pair of struts (second struts) 521 (hereinafter also referred to as "521a to 521b") and one strut (second strut) 521 arranged with a clearance (a hole 523) from the pair of struts 521. Moreover, the inner cell 522 includes, in the annular direction CD2, two struts 521 arranged with a clearance (the hole 523) therebetween so as to face each other. The strut 521 arranged apart from the pair of struts 521 in the annular direction CD1 in a certain inner cell 522 is one strut 521a of the pair of struts 521 in another inner cell 522 adjacent to the certain inner cell 522 in the annular direction CD1.

The pair of struts 521a to 521b and one strut 521 arranged in the annular direction CD1 form the long sides of the inner cell 522. Two struts 521 arranged in the annular direction CD2 form the short sides of the inner cell 522. The inner cell 522 is configured, when opened in a planar shape, such that the long-side struts 521a to 521b, 521 and the short-side struts 521 are diagonally coupled substantially in the form of a parallelogram.

In the inner cell 522, the hole 523 is formed. In each inner cell 522 arranged along the annular direction CD1, the pair of struts 521a to 521b and the struts 521 extending along the annular direction CD1 are connected at substantially S-shaped second intersections 524. The second intersection 524 deforms so as to stretch in the radial direction RD when the expanded stent 1E is bent substantially in the U-shape (see FIG. 7). Thus, the inner cells 522 spread in the radial direction RD can be more flexibly bent. As shown in FIG. 16B, in the second stent body 520, the second intersections 524 are arranged in parallel in the circumferential direction OD.

As shown in FIG. 16C, in the stent 1E, the first stent body 510 and the second stent body 520 overlap with each other such that the second intersection 524 between the inner cells 522 (of the second stent body 520) is arranged in the hole 513 of the outer cell 512 (of the first stent body 510). Specifically, in a configuration in which the second intersection 524 between the inner cells 522 is arranged in the hole 513 of the outer cell 512, the first stent body 510 and the second stent body 520 overlap with each other such that one second intersection 524 between the inner cells 522 is arranged in one hole 513 of the outer cell 512. Moreover, in the stent 1E, the first intersections 514 of the outer cells 512 and the second intersections 524 of the inner cells 522 are arranged in parallel in the circumferential direction OD, and are arranged alternately. In a case where each stent body is configured with the cell shape shown in FIGS. 16A and 16B, the mesh patterns of the stent bodies overlap with each other as described above so that the density of the mesh pattern is increased over the entire stent and the surface area of the stent 1E can be more increased.

Further, in the stent 1E of the sixth embodiment, the outer cells 512 of the first stent body 510 are connected, in the annular direction CD1, to each other at the substantially S-shaped first intersections 514. Similarly, the inner cells 522 of the second stent body 520 are connected, in the annular direction CD1, to each other at the substantially S-shaped second intersections 524. The first intersections 514 of the first stent body 510 and the second intersections 524 of the second stent body 520 are arranged in parallel in the circumferential direction OD, as shown in FIG. 16C. According to the present configuration, the outer cells 512 and the inner cells 522 spread in the circumferential direction OD can be more flexibly bent when the expanded stent 1E is bent substantially in the U-shape (see FIG. 7), and therefore, the shape followability of the stent 1E can be more enhanced.

### (Seventh Embodiment)

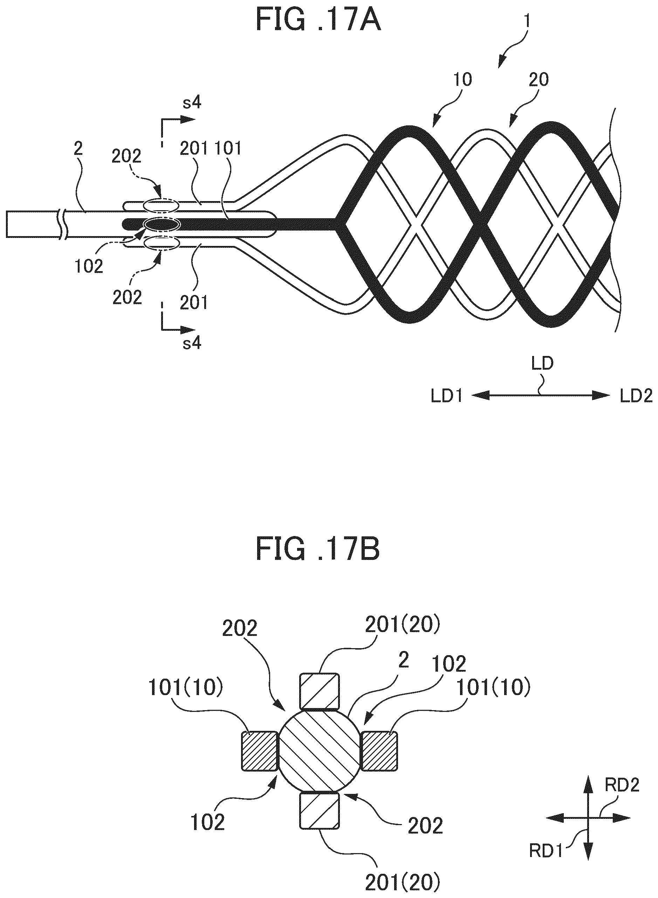

FIG. 17A is a side view schematically showing a configuration in which a proximal side end portion of a stent 1 and a push wire 2 are connected in a first connection form. FIG. 17B is a sectional view along an s4-s4 line of FIG. 17A. Note that in description of a seventh embodiment and later-described eighth and ninth embodiments, the stent 1 (see FIG. 1) of the first embodiment will be described as an example of a stent for describing a connection form, but the connection form may be applied to stents of other embodiments. Moreover, in each figure described below, the configuration of the stent 1 is simplified.

As shown in FIG. 17A, a proximal side LD1 end portion 101 of the first stent body 10 is connected, at a connection portion 102, to the push wire 2. A proximal side LD1 end portion 201 of the second stent body 20 is connected, at a connection portion 202, to the push wire 2. In the first connection form, the connection portion 102 of the first stent body 10 and the connection portion 202 of the second stent body 20 are provided at the same position in the axial direction LD of the push wire 2. Positions at which the connection portion 102 of the first stent body 10 and the connection portion 202 of the second stent body 20 are connected by, e.g., welding and the areas thereof are schematically shown.

As shown in FIG. 17B, the connection portions 102 of the first stent body 10 and the connection portions 202 of the second stent body 20 are provided at equal intervals in the radial direction (the direction perpendicular to the axial direction LD) of the push wire 2. Note that FIG. 17B shows the example where the connection portions 102 of the first stent body 10 and the connection portions 202 of the second stent body 20 are provided at an interval of 90 degrees in the circumferential direction, but the present invention is not limited to this example.

### (Eighth Embodiment)

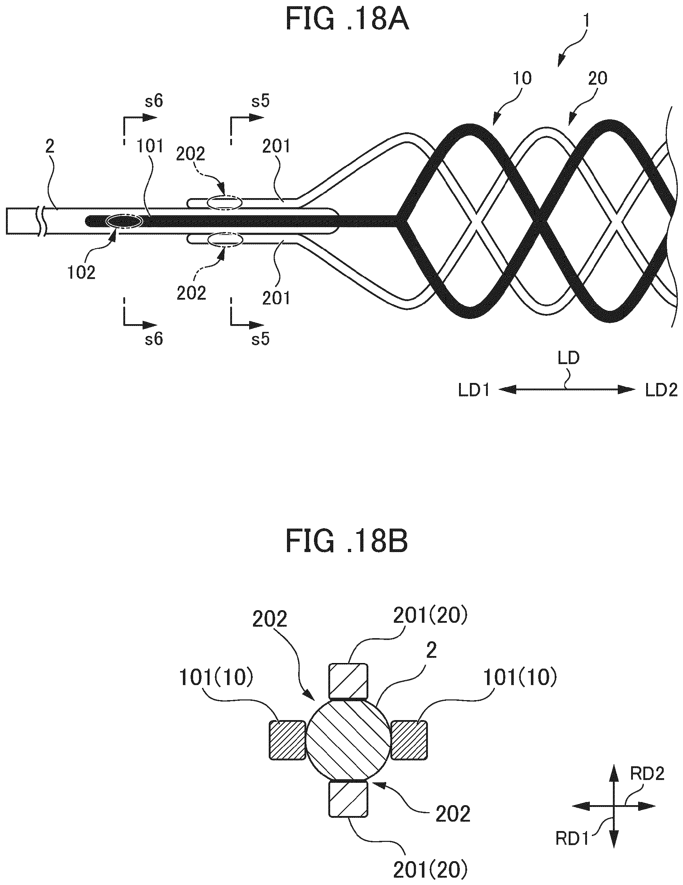

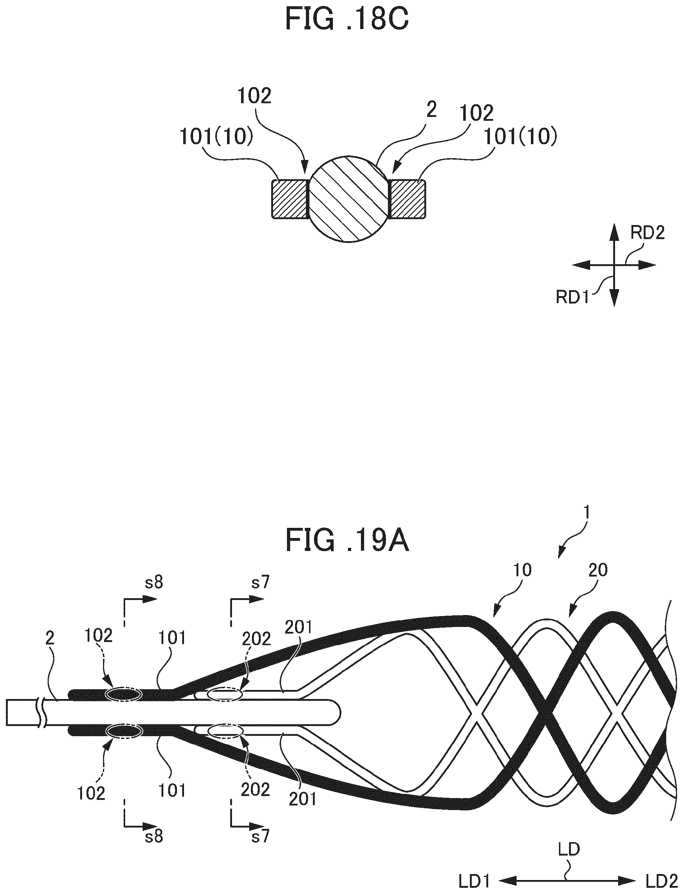

FIG. 18A is a side view schematically showing a configuration in which the proximal side end portion of the stent 1 and the push wire 2 are connected in a second connection form. FIG. 18B is a sectional view along an s5-s5 line of FIG. 18A. FIG. 18C is a sectional view along an s6-s6 line of FIG. 18A. For the sake of easy recognition of the position of each connection portion in FIGS. 18B and 18C, the up-down direction in the figure will be referred to as a first radial direction RD1, and the right-left direction, which is perpendicular to the first radial direction RD1, in the figure will be referred to as a second radial direction RD2. Note that the first radial direction RD1 and the second radial direction RD2 are not limited to the up-down direction and the right-left direction in the drawing.

As shown in FIG. 18A, the proximal side LD1 end portion 101 of the first stent body 10 and the proximal side LD1 end portion 201 of the second stent body 20 are connected at different positions in the axial direction LD of the push wire 2. Specifically, the end portion 101 of the first stent body 10 extends from the distal side LD2 to proximal side LD1 along the side surface of the push wire 2. Moreover, the end portion 101 of the first stent body 10 is connected at the connection portion 102 on the proximal side LD1 with respect to the connection portion 202 of the second stent body 20. On the other hand, the end portion 201 of the second stent body 20 is positioned on the distal side LD2 of the push wire 2. Moreover, the end portion 201 of the second stent body 20 is connected at the connection portion 202 on the distal side LD2 with respect to the end portion 101 of the first stent body 10.

The connection portions 102 of the first stent body 10 and the connection portions 202 of the second stent body 20 are provided at equal intervals as viewed in the axial direction LD of the push wire 2. For example, as shown in FIG. 18B, the connection portions 202 of the second stent body 20 are provided at an interval of 180 degrees in the first radial direction RD1. In FIG. 18B, the end portion 101 of the first stent body 10 contacts the side surface of the push wire 2, but is not connected at the connection portion 102. Moreover, as shown in FIG. 18C, the connection portions 102 of the first stent body 10 are provided at an interval of 180 degrees in the second radial direction RD2.

According to the configuration of the present embodiment, in the axial direction LD of the push wire 2, the end portion 101 of the first stent body 10 and the end portion 201 of the second stent body 20 are not connected at the same position. Thus, a defect such as distortion of the push wire 2 due to heat upon welding can be reduced. Note that in the axial direction LD of the push wire 2, the positions of the connection portion 102 of the first stent body 10 and the connection portion 202 of the second stent body 20 may be switched such that the connection portion 102 of the first stent body 10 is provided on the distal side LD2 with respect to the connection portion 202 of the second stent body 20.

### (Ninth Embodiment)

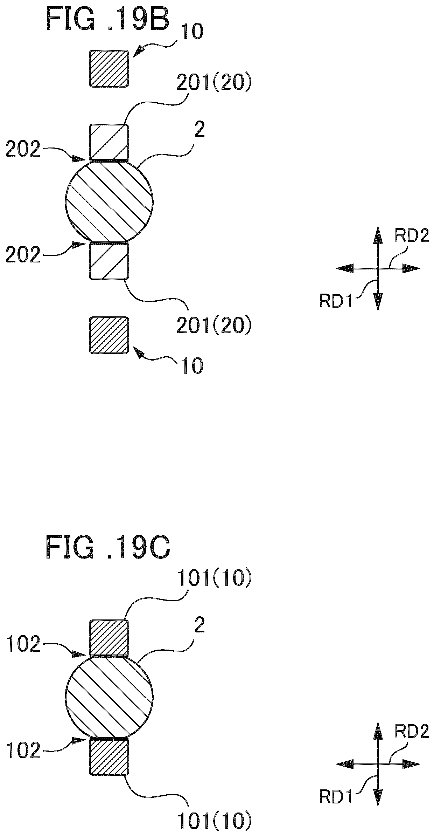

FIG. 19A is a side view schematically showing a configuration in which the proximal side end portion of the stent 1 and the push wire 2 are connected in a third connection form. FIG. 19B is a sectional view along an s7-s7 line of FIG. 19A. FIG. 19C is a sectional view along an s8-s8 line of FIG. 19A. For the sake of easy recognition of the position of each connection portion in FIGS. 19B and 19C, the up-down direction in the figure will be referred to as a first radial direction RD1, and the right-left direction, which is perpendicular to the first radial direction RD1, in the figure will be referred to as a second radial direction RD2, as in the eighth embodiment.

As shown in FIG. 19A, the end portion 101 of the first stent body 10 and the end portion 201 of the second stent body 20 are connected at different positions in the axial direction LD of the push wire 2. Specifically, the end portion 101 of the first stent body 10 extends from the distal side LD2 to the proximal side LD1 over the end portion 201 of the second stent body 20. Moreover, the end portion 101 of the first stent body 10 is connected at the connection portion 102 on the proximal side LD1 with respect to the end portion 201 of the second stent body 20. On the other hand, the end portion 201 of the second stent body 20 is positioned on the distal side LD2 of the push wire 2. Moreover, the end portion 201 of the second stent body 20 is connected at the connection portion 202 on the distal side LD2 with respect to the end portion 101 of the first stent body 10.

The connection portions 102 of the first stent body 10 and the connection portions 202 of the second stent body 20 are provided at equal intervals as viewed in the axial direction LD of the push wire 2. For example, as shown in FIG. 19B, the connection portions 202 of the second stent body 20 are provided at an interval of 180 degrees in the first radial direction RD1. Moreover, as shown in FIG. 19C, the connection portions 102 of the first stent body 10 are provided at an interval of 180 degrees in the first radial direction RD1, as in the connection portion 202 of the second stent body 20.

Note that in the axial direction LD of the push wire 2, the positions of the connection portion 102 of the first stent body 10 and the connection portion 202 of the second stent body 20 may be switched such that the connection portion 102 of the first stent body 10 is provided on the distal side LD2 with respect to the connection portion 202 of the second stent body 20. Moreover, in FIG. 19B, the connection portions 202 of the second stent body 20 may be provided at an interval of 180 degrees in the second radial direction RD2, and the connection portions 102 of the first stent body 10 may be provided at an interval of 180 degrees in the second radial direction RD2.

### (Tenth Embodiment)

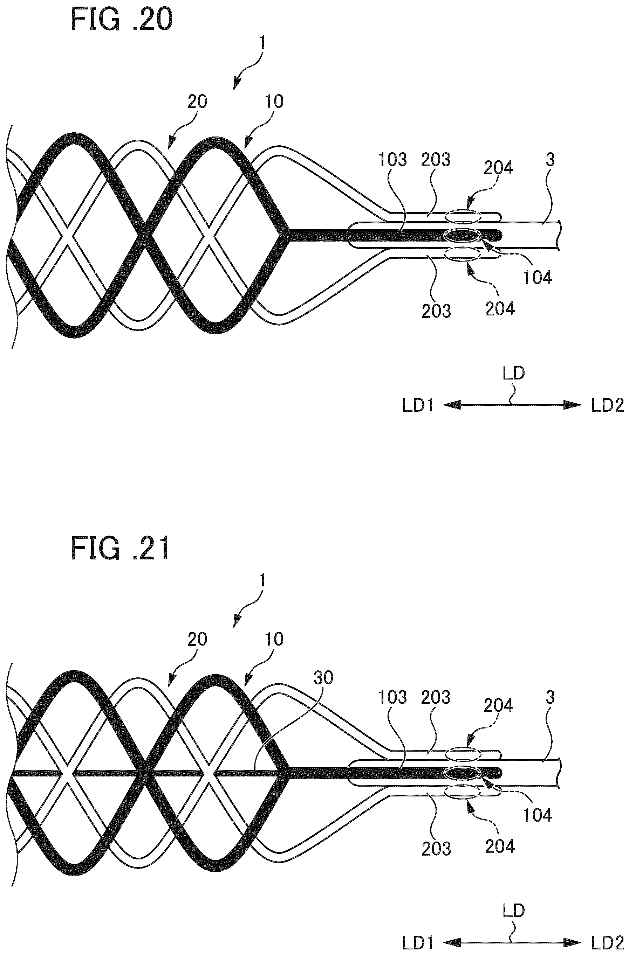

The connection forms of the proximal side end portion of the stent 1 and the push wire 2 as described above in the seventh to ninth embodiments are also applicable to a connection form of the distal side LD2 of the stent 1 and the distal end shaft 3. FIG. 20 is a side view schematically showing a configuration in which the distal side end portion of the stent 1 and the distal end shaft 3 are connected in the first connection form (the seventh embodiment).

As shown in FIG. 20, a distal side LD2 end portion 103 of the first stent body 10 is connected, at a connection portion 104, to the distal end shaft 3. A distal side LD2 end portion 203 of the second stent body 20 is connected, at a connection portion 204, to the distal end shaft 3. The connection portion 104 of the first stent body 10 and the connection portion 204 of the second stent body 20 are provided at the same position in the axial direction LD of the distal end shaft 3. Although not shown in the figure, the connection portions 104 of the first stent body 10 and the connection portions 204 of the second stent body 20 are provided at equal intervals as viewed in the axial direction LD of the distal end shaft 3 (see, e.g., FIG. 17B).

FIG. 21 is a side view schematically showing another configuration in which the distal side end portion of the stent 1 and the distal end shaft 3 are connected in the first connection form (see FIG. 20). As shown in FIG. 21, it may be configured such that a metal wire 30 having a high radiopacity is inserted into the stent 1. A distal side LD2 end portion of the metal wire 30 is connected to the distal end shaft 3. Although not shown in the figure, a proximal side LD1 end portion of the metal wire 30 is connected to the push wire 2 (see FIG. 1).

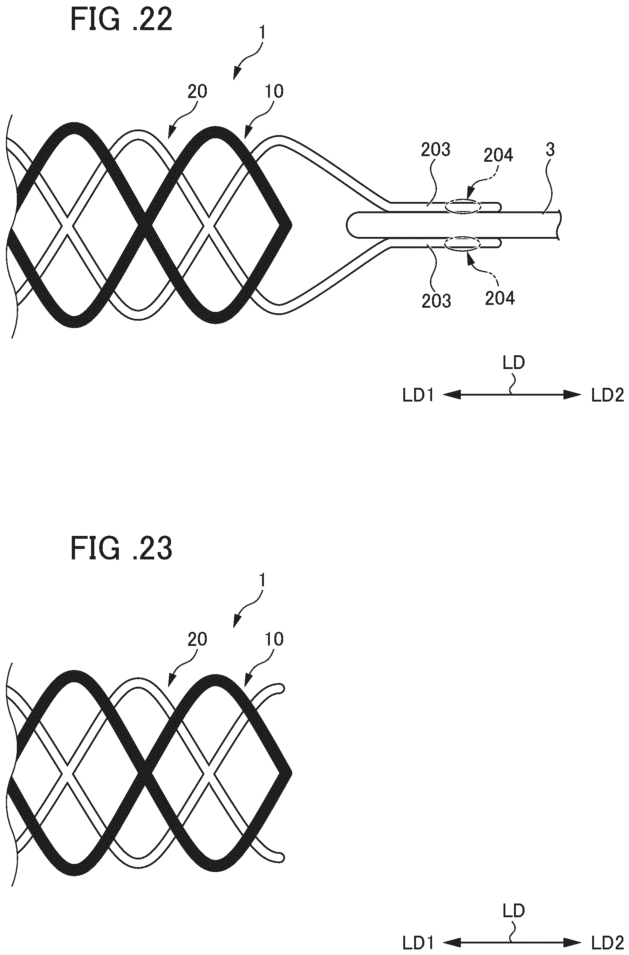

FIG. 22 is a side view schematically showing a configuration in which the distal side end portion of the stent 1 and the distal end shaft 3 are connected in a fourth connection form. As shown in FIG. 22, the distal side LD2 end portion 203 of the second stent body 20 is connected, at the connection portion 204, to the distal end shaft 3. On the other hand, the distal side LD2 end portion of the first stent body 10 is not connected to the distal end shaft 3. That is, in the fourth connection form, only the distal side LD2 end portion 203 of the second stent body 20 on the distal side of the stent 1 is connected to the distal end shaft 3. Note that it may be configured such that in the fourth connection form shown in FIG. 20, only the distal side LD2 end portion 103 of the first stent body 10 on the distal side of the stent 1 is connected to the distal end shaft 3.

FIG. 23 is a side view schematically showing another configuration of the stent 1 on the distal side thereof. As shown in FIG. 23, it may be configured such that the first stent body 10 and the second stent body 20 are opened on the distal side LD2. Note that although not shown in the figure, the proximal side LD1 end portion of the first stent body 10 and the proximal side LD1 end portion of the second stent body 20 are connected to the push wire 2 (see FIG. 1) in the configuration shown in FIG. 23.

### (Eleventh Embodiment)

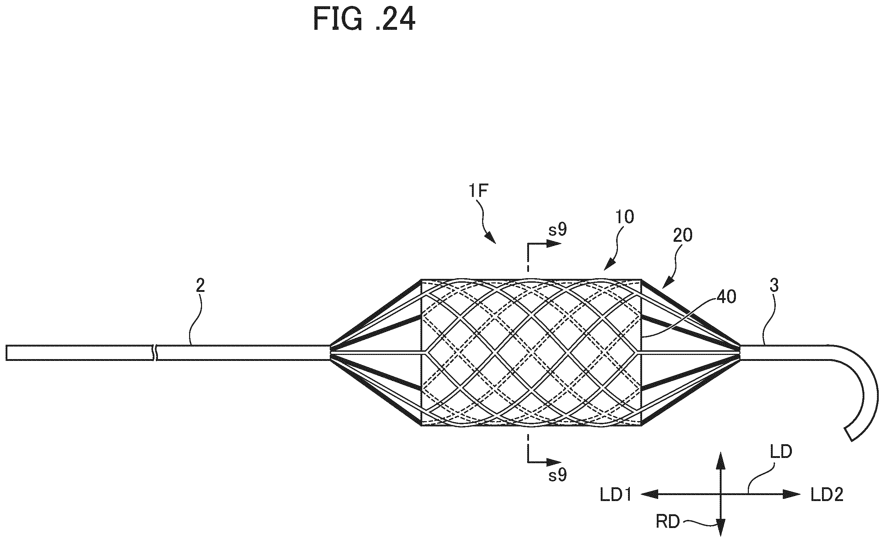

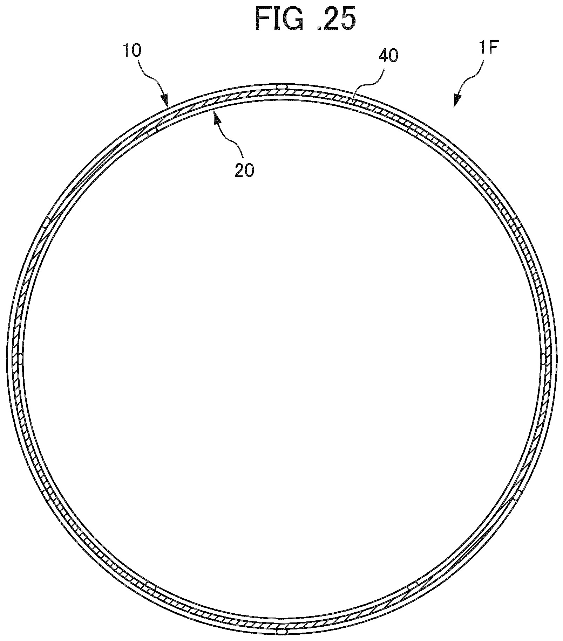

FIG. 24 is a schematic side view of a stent 1F of an eleventh embodiment. FIG. 25 is a sectional view along an s9-s9 line of FIG. 24. In description and drawings of the eleventh embodiment, the same reference numerals as those of the first embodiment are used to represent members etc. similar to those of the first embodiment, and overlapping description thereof will be omitted. As shown in FIGS. 24 and 25, the stent 1F of the eleventh embodiment includes a coating film 40 between a first stent body 10 and a second stent body 20. The coating film 40 is provided substantially in a cylindrical shape, and extends along the axial direction LD of the stent 1F. For the coating film 40, a material such as PTFE or ePTFE may be used, for example. The thickness of the coating film 40 is, for example, about 0.05 to 0.2 mm.

The coating film 40 is provided between the first stent body 10 and the second stent body 20 so that infarction in a distal side blood vessel due to, e.g., leakage of plaque or blood clot through a clearance among struts (see FIGS. 3A to 3C) can be reduced. The coating film 40 is not necessarily provided between the first stent body 10 and the second stent body 20, but may be provided outside the first stent body 10.

The coating film 40 may contain a medical agent. The coating film 40 containing the medical agent indicates that the coating film 40 releasably carries the medical agent so as to dissolve out the medical agent. The medical agent is not limited, and for example, may include the medical agents described as examples in the configuration of the stent 1 of the first embodiment containing the medical agent. The coating film 40 may be made of an antithrombogenic material having a blood coagulation inhibition function.

### (Twelfth Embodiment)

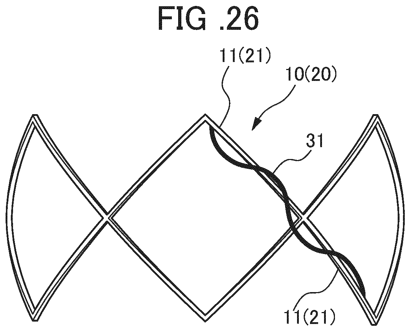

Next, an embodiment in which a strand having a high radiopacity is wound around a stent will be described. In the present embodiment, the stent 1 (see FIG. 1) of the first embodiment will be described as an example of the stent around which the strand having the high radiopacity is wound, but this configuration may be applied to stents of other embodiments. FIG. 26 is a schematic view showing an example where a strand 31 (hereinafter also referred to as a "strand 31") having a high radiopacity is sparsely wound around the strut 11 of the first stent body 10. In the example shown in FIG. 26, the strand 31 may be wound around all the struts 11 of the first stent body 10, or be wound around only some of the struts 11.

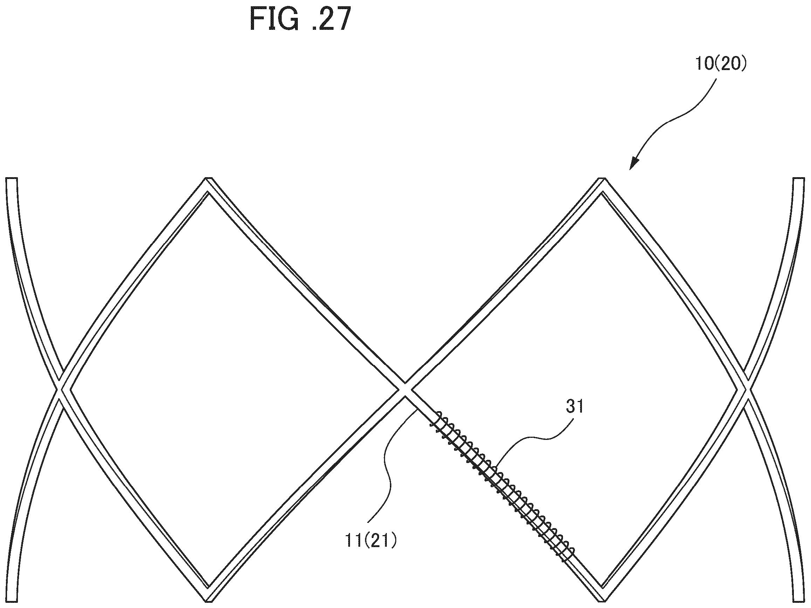

FIG. 27 is a schematic view showing an example where the strand 31 having the high radiopacity is densely wound (in a coil shape) around the strut 11 of the first stent body 10. In the example shown in FIG. 27, the strand 31 may be wound around all the struts 11 of the first stent body 10, or be wound around only some of the struts 11.

Note that in the examples shown in FIGS. 26 and 27, the strand 31 may be wound around the struts 21 of the second stent body 20 in a similar form. The strand 31 may be wound around both the first stent body 10 and the second stent body 20, or be wound around only either one of the first stent body 10 or the second stent body 20.

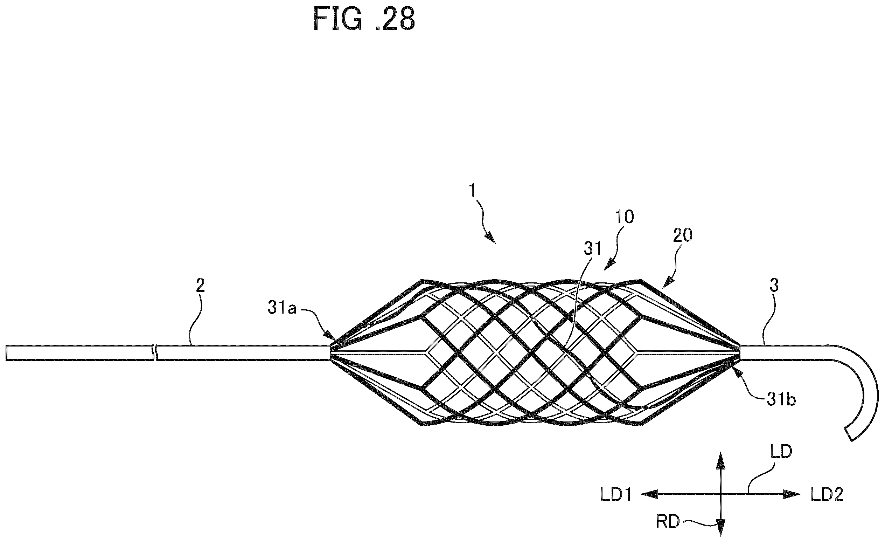

FIG. 28 is a schematic view showing an example where the strand 31 having the high radiopacity is wound around the stent 1 in a first form. As shown in FIG. 28, in the first form, the strand 31 is wound around the second stent body 20, which is positioned inside the stent 1, in a spiral shape. One end portion 31a of the strand 31 is connected to the proximal side LD1 of the second stent body 20. The other end portion 31b of the strand 31 is connected to the distal side LD2 of the second stent body 20. The strand 31 is wound around the second stent body 20 in the spiral shape so that the visibility of the stent 1 expanded in a blood vessel on an X-ray transparent image can be enhanced. Note that a plurality of strands 31 may be wound. In a case where the plurality of strands 31 is wound, it can be checked whether or not the stent 1 expanded in the blood vessel is partially opened.

The strand 31 may be connected to the strut 21 (see FIG. 3B) of the second stent body 20 by, e.g., welding. The strand 31 is not necessarily connected to the second stent body 20, but may be connected to the first stent body 10 positioned outside the stent 1 or be connected to different stent bodies. For example, it may be configured such that one end portion of the strand 31 is connected to the proximal side LD1 of the second stent body 20 and the other end portion is connected to the distal side LD2 of the first stent body 10.

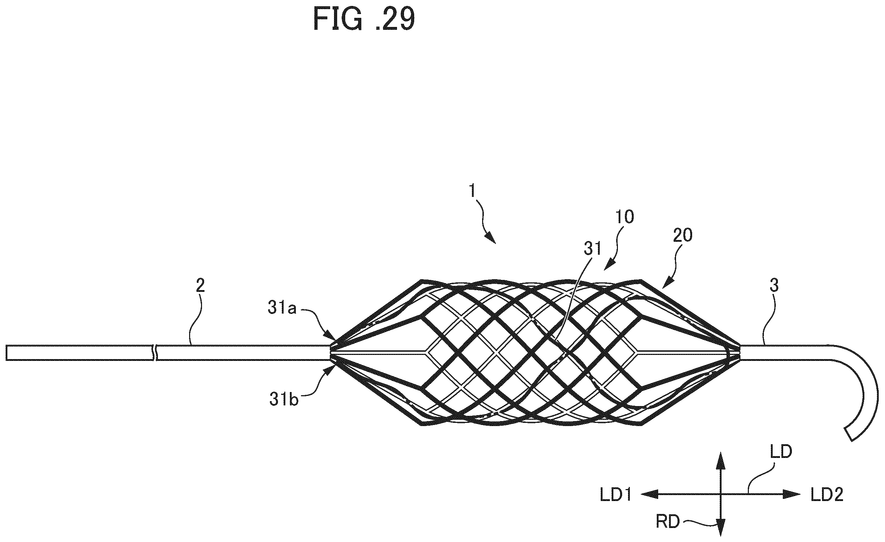

FIG. 29 is a schematic view showing an example where the strand 31 having the high radiopacity is wound around the stent 1 in a second form. As shown in FIG. 29, in the second form, the strand 31 is wound around the second stent body 20 in a spiral shape so as to extend back and forth between both end portions of the second stent body 20. One end portion of the strand 31 is connected to the proximal side LD1 of the second stent body 20. The strand 31 is wound around the second stent body 20 in the spiral shape from the proximal side LD1 to the distal side LD2 of the stent 1. The strand 31 is folded back at the distal side LD2 end portion of the second stent body 20, and is wound around the second stent body 20 in the spiral shape from the distal side LD2 to the proximal side LD1 of the stent 1. The other end portion of the strand 31 is connected to the proximal side LD1 of the second stent body 20. In the second form shown in FIG. 29, advantageous effects similar to those of the above-described first embodiment can be obtained.

In the present form, the strand 31 may be connected to the strut 21 of the second stent body 20 by, e.g., welding. The strand 31 is not necessarily connected to the second stent body 20, but may be connected to the first stent body 10 positioned outside the stent 1 or be connected to different stent bodies. For example, it may be configured such that one end portion of the strand 31 is connected to the proximal side LD1 of the second stent body 20 and the other end portion is connected to the distal side LD2 of the first stent body 10.

### (Thirteenth Embodiment)

Next, a stent 1G of a thirteenth embodiment will be described. The stent 1G of the thirteenth embodiment is different from that of the first embodiment in the cell shapes of first and second stent bodies. Other configurations of the stent 1G of the thirteenth embodiment are the same as those of the first embodiment. In description below and the drawings, the same reference numerals are used as end numerals (last two digits) of elements having functions similar to those of the first embodiment, and overlapping description thereof will be omitted as necessary.

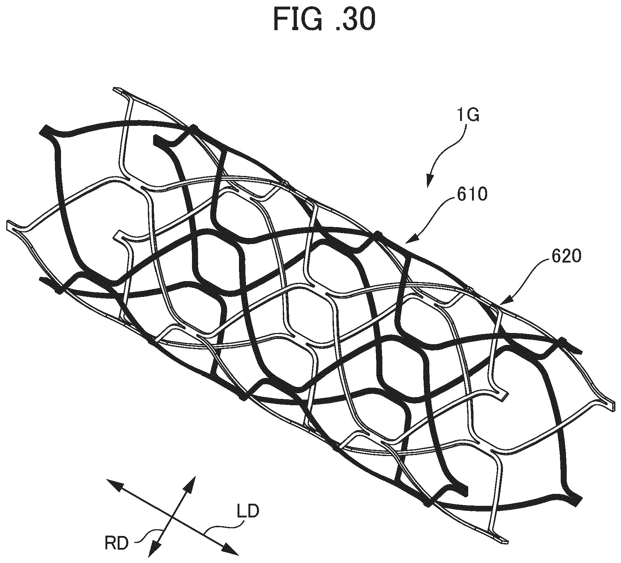

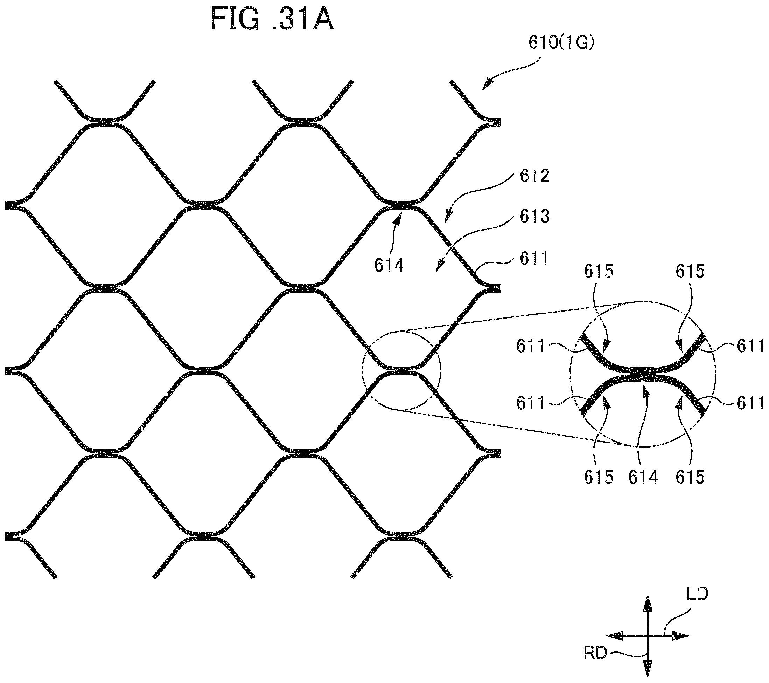

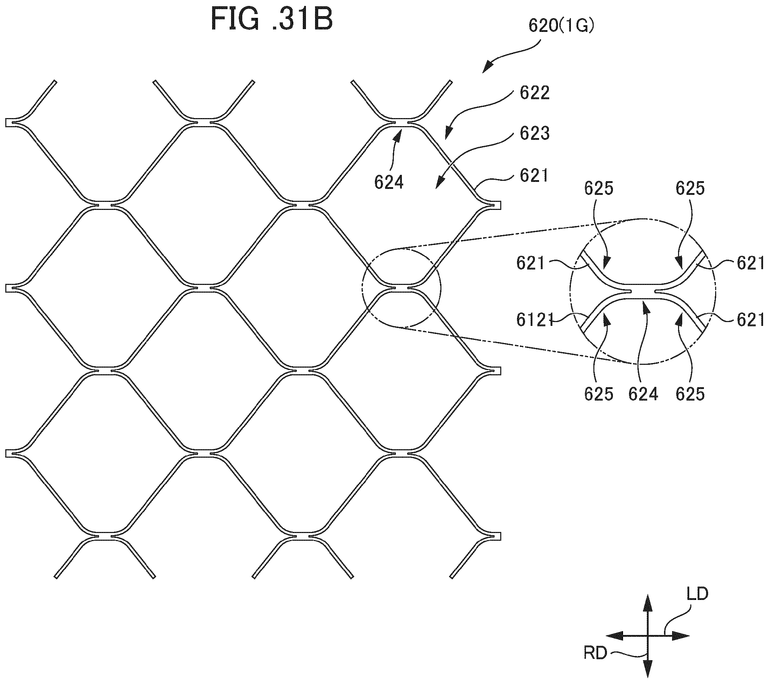

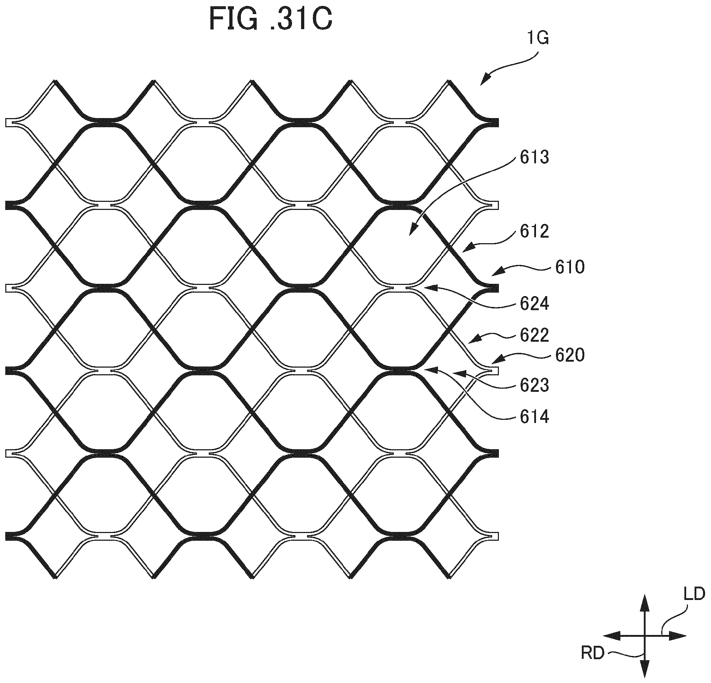

FIG. 30 is a schematic perspective view of the stent 1G of the thirteenth embodiment. FIG. 31A is a development view showing a state in which part of a first stent body 610 of the thirteenth embodiment is virtually opened in a planar shape. FIG. 31B is a development view showing a state in which part of a second stent body 620 of the thirteenth embodiment is virtually opened in a planar shape. FIG. 31C is a development view showing a state in which part of the stent 1G of the thirteenth embodiment is virtually opened in a planar shape.

As shown in FIG. 30, the stent 1G of the thirteenth embodiment includes the first stent body 610 and the second stent body 620. The first stent body 610 is a substantially cylindrical structure arranged outside the stent 1G. The second stent body 620 is a substantially cylindrical structure arranged inside the first stent body 610. The stent 1G has such a double-layer structure in which the second stent body 620 is inserted into the first stent body 610. Note that FIG. 30 does not show the push wire 2 and the distal end shaft 3 shown in FIG. 1.

The first stent body 610 is configured, as shown in FIG. 31A, such that a plurality of outer cells (first cells) 612 including struts 611 arranged in a frame shape spreads in a radial direction (circumferential direction) RD. In the first stent body 610, the plurality of outer cells 612 spread in the radial direction RD is continuously arranged in an axial direction LD. That is, the first stent body 610 has such a mesh pattern that the plurality of outer cells 612 spreads in the radial direction RD and is continuously arranged in the axial direction LD. In the outer cell 612, a hole 613 is formed. Adjacent ones of the outer cells 612 in the radial direction RD are connected at an intersection 614.

The intersection 614 is a portion where the struts 611 of adjacent four of the outer cells 612 are connected to each other. The intersection 614 has a substantially rectangular shape elongated in the axial direction LD. The struts 611 are each connected to four corners of the intersection 614. Each strut 611 has a curved portion 615 at the portion connected to the intersection 614. Thus, as compared to the intersecting point 14 (the outer cell 12) of the first embodiment, the strut 611 is in a shape elongated in the axial direction LD at the intersection 614 (the outer cell 612) of the present embodiment. Thus, when the expanded stent 1G is bent substantially in a U-shape (see FIG. 7), the struts 611 connected to the intersection 614 are independently deformable. Thus, the outer cells 612 spread in the radial direction RD can be more flexibly bent. As described above, the outer cells 612 spread in the radial direction RD can be more flexibly bent, and therefore, the first stent body 610 has excellent shape followability and diameter reducibility.

The second stent body 620 is configured, as shown in FIG. 31B, such that a plurality of inner cells (second cells) 622 including struts 621 arranged in a frame shape spreads in the radial direction (circumferential direction) RD. In the second stent body 620, the plurality of inner cells 622 spread in the radial direction RD is continuously arranged in the axial direction LD. That is, the second stent body 620 has such a mesh pattern that the plurality of inner cells 622 spreads in the radial direction RD and is continuously arranged in the axial direction LD. In the inner cell 622, a hole 623 is formed. Adjacent ones of the inner cells 622 in the radial direction RD are connected at an intersection 624.

The intersection 624 is a portion where the struts 621 of adjacent four of the inner cells 622 are connected to each other. The intersection 624 has a substantially rectangular shape elongated in the axial direction LD. The struts 621 are each connected to four corners of the intersection 624. Each strut 621 has a curved portion 625 at the portion connected to the intersection 624. Thus, as compared to the intersecting point 24 (the inner cell 22) of the first embodiment, the strut 621 is in a shape elongated in the axial direction LD at the intersection 624 (the inner cell 622) of the present embodiment. Thus, when the expanded stent 1G is bent substantially in the U-shape, the struts 621 connected to the intersection 624 are independently deformable in the radial direction RD. Thus, the inner cells 622 spread in the radial direction RD can be more flexibly bent. As described above, the inner cells 622 spread in the radial direction RD can be more flexibly bent, and therefore, the second stent body 620 has excellent shape followability and diameter reducibility.

As shown in FIGS. 31A and 31B, in the stent 1G of the thirteenth embodiment, the outer cell 612 forming the first stent body 610 and the inner cell 622 forming the second stent body 620 have the same size, shape, and arrangement, as one example. That is, in the thirteenth embodiment, the mesh pattern of the first stent body 610 shown in FIG. 13A and the mesh pattern of the second stent body 620 shown in FIG. 31B are substantially the same pattern. Note that the mesh pattern of the first stent body 610 and the mesh pattern of the second stent body 620 may be different from each other.

As shown in FIG. 31C, in the stent 1G, the first stent body 610 and the second stent body 620 overlap with each other such that the intersection 624 between the inner cells 622 (of the second stent body 620) is arranged in the hole 613 of the outer cell 612 (of the first stent body 610). Specifically, in a configuration in which the intersection 624 between the inner cells 622 is arranged in the hole 613 of the outer cell 612, the first stent body 610 and the second stent body 620 overlap with each other such that one intersection 624 between the inner cells 622 is arranged in one hole 613 of the outer cell 612. The mesh patterns of the stent bodies overlap with each other as described above, and therefore, the density of the mesh pattern is increased over the entire stent. Thus, the surface area of the stent 1G can be increased. The stent 1G of the present embodiment is configured such that the intersection 614 among the outer cells 612 has the configuration shown in FIG. 31A and the intersection 624 among the inner cells 622 has the configuration shown in FIG. 31B. Thus, the stent 1G has excellent shape followability and diameter reducibility. Moreover, the stent 1G is configured such that the outer cells 612 and the inner cells 622 have the configuration described above, and therefore, produces effects that the narrowed stent 1G is easily sheathed in a catheter and the stent 1G expanded in a blood vessel is easily resheathed in a catheter.

### (Fourteenth Embodiment)

Next, a stent 1H of a fourteenth embodiment will be described. The stent 1H of the fourteenth embodiment is different from that of the fourth embodiment (see FIGS. 12A and 12B) in the cell shapes of first and second stent bodies. Other configurations of the stent 1H of the fourteenth embodiment are the same as those of the fourth embodiment. Thus, in the fourteenth embodiment, the entirety of the stent 1H is not shown in the figure. In description and drawings of the fourteenth embodiment, the same reference numerals are used to represent elements having functions similar to those of the fourth embodiment, and overlapping description thereof will be omitted as necessary. In the fourteenth embodiment, annular directions of cells being connected diagonally to a radial direction (circumferential direction) RD will be referred to as annular directions CD1, CD2.

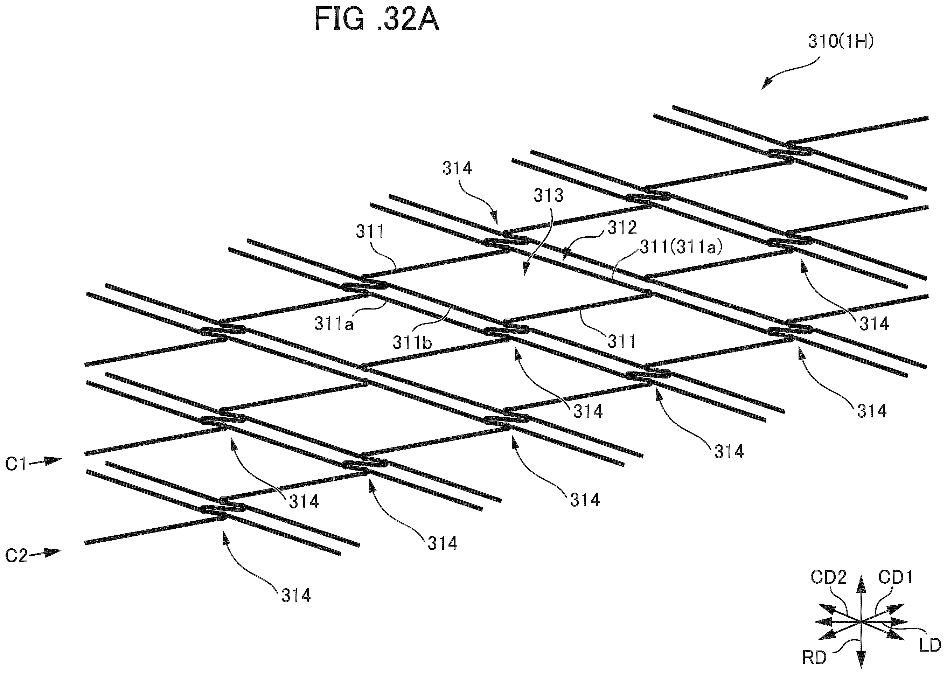

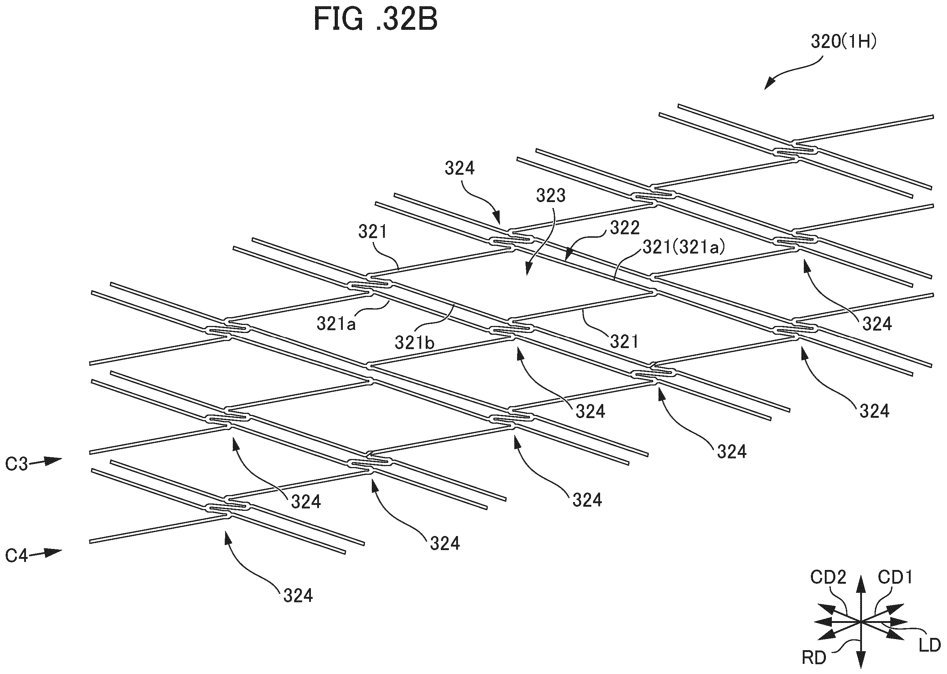

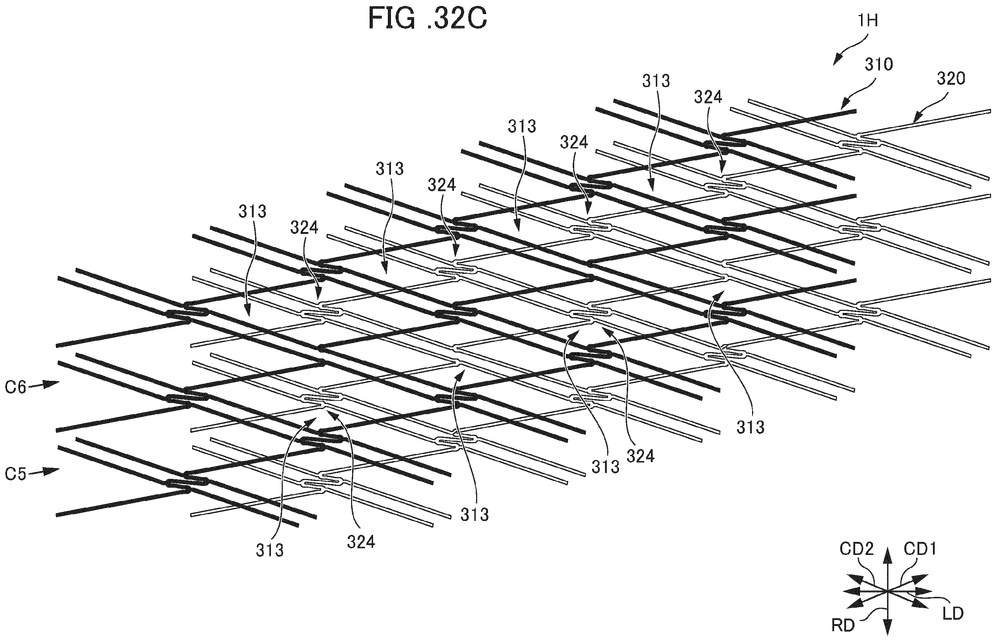

FIG. 32A is a development view showing a state in which a first stent body 310 of the fourteenth embodiment is virtually opened in a planar shape. FIG. 32B is a development view showing a state in which a second stent body 320 of the fourteenth embodiment is virtually opened in a planar shape. FIG. 32C is a development view showing a state in which the stent 1H of the fourteenth embodiment is virtually opened in a planar shape.

The first stent body 310 of the fourteenth embodiment is different from that of the fourth embodiment in arrangement of first intersections 314. As shown in FIG. 32A, the first stent body 310 of the fourteenth embodiment includes, in the annular direction CD1, a line C1 in which the first intersections 314 are arranged in every other position and a line C2 in which the first intersection 314 is arranged between each adjacent ones of the cells. The lines C1 and the lines C2 are alternately arranged in the annular direction CD2. Other configurations of the first stent body 310 of the fourteenth embodiment are the same as those of the fourth embodiment.

The second stent body 320 of the fourteenth embodiment is different from that of the fourth embodiment in arrangement of second intersections 324. As shown in FIG. 32B, the second stent body 320 of the fourteenth embodiment includes, in the annular direction CD1, a line C3 in which the second intersections 324 are arranged in every other position and a line C4 in which the second intersection 324 is arranged between each adjacent ones of the cells. The lines C3 and the lines C4 are alternately arranged in the annular direction CD2. Other configurations of the second stent body 320 of the fourteenth embodiment are the same as those of the fourth embodiment.

In the stent 1H of the fourteenth embodiment, the plurality of outer cells 312 forming the first stent body 310 and the plurality of inner cells 322 forming the second stent body 320 have the same size, shape, and arrangement, as one example. That is, in the fourteenth embodiment, the mesh pattern of the first stent body 310 shown in FIG. 32A and the mesh pattern of the second stent body 320 shown in FIG. 32B are substantially the same pattern. Note that the mesh pattern of the first stent body 310 and the mesh pattern of the second stent body 320 may be different from each other.

As shown in FIG. 32C, in the stent 1H of the fourteenth embodiment, in a line C5, the cells in each of which the second intersection 324 of the second stent body 320 is arranged in the hole 313 of the first stent body 310 and the cells in each of which no second intersection 324 is present in the hole 313 are alternately arranged in the annular direction CD1. In a line C6, the second intersection 324 of the second stent body 320 is arranged in the hole 313 of the first stent body 310 in the annular direction CD1. Moreover, in the stent 1H of the fourteenth embodiment, the first stent body 310 and the second stent body 320 overlap with each other such that the lines C5 and the lines C6 are alternately arranged in the annular direction CD2.

In a case where each stent body is configured, as in the fourteenth embodiment, with the cell shape shown in FIGS. 32A and 32B, the mesh patterns of the stent bodies overlap with each other as described above so that the density of the mesh pattern is increased over the entire stent and the surface area of the stent 1H can be more increased. In the stent 1H of the fourteenth embodiment, the outer cells 312 and the inner cells 322 spread in the radial direction RD can be more flexibly bent as in the stent 1C of the fourth embodiment when the expanded stent 1H is bent substantially in a U-shape (see FIG. 7), and therefore, the shape followability of the stent 1H can be more enhanced.

### (Fifteenth Embodiment)

Next, a stent 1J of a fifteenth embodiment will be described. The stent 1J of the fifteenth embodiment is different from that of the fourth embodiment (see FIGS. 12A and 12B) in the cell shapes of first and second stent bodies. Other configurations of the stent 1J of the fifteenth embodiment are the same as those of the fourth embodiment. Thus, in the fifteenth embodiment, the entirety of the stent 1J is not shown in the figure. In description and drawings of the fifteenth embodiment, the same reference numerals are used to represent elements having functions similar to those of the fourth embodiment, and overlapping description thereof will be omitted as necessary. In the fifteenth embodiment, annular directions of cells being connected diagonally to a radial direction (circumferential direction) RD will be referred to as annular directions CD1, CD2.

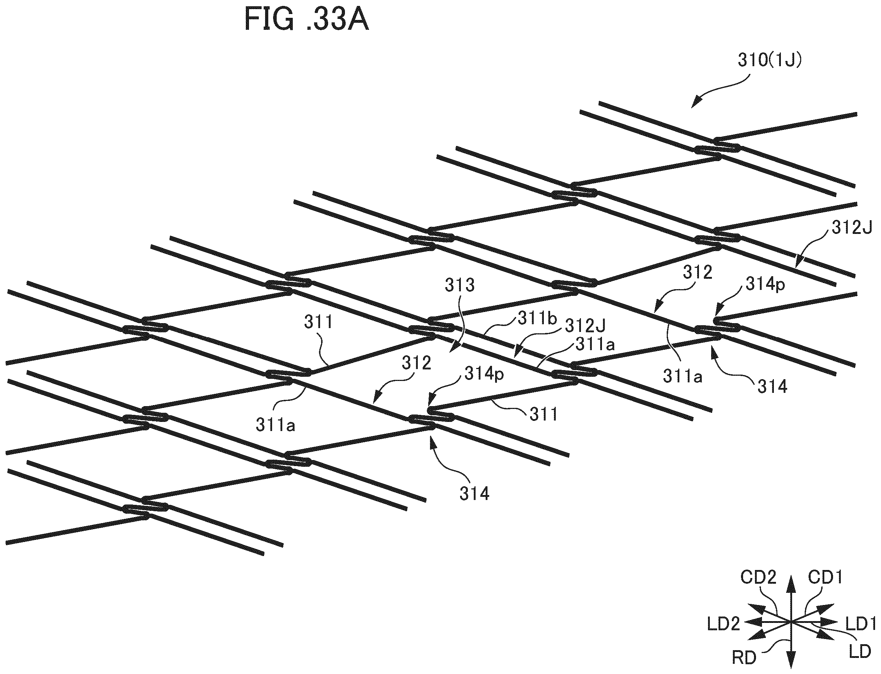

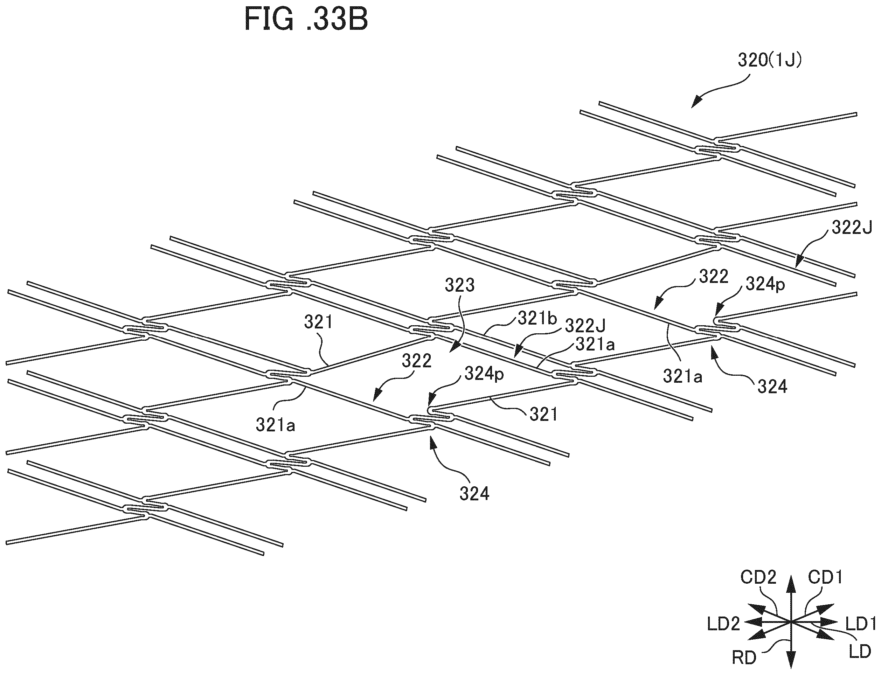

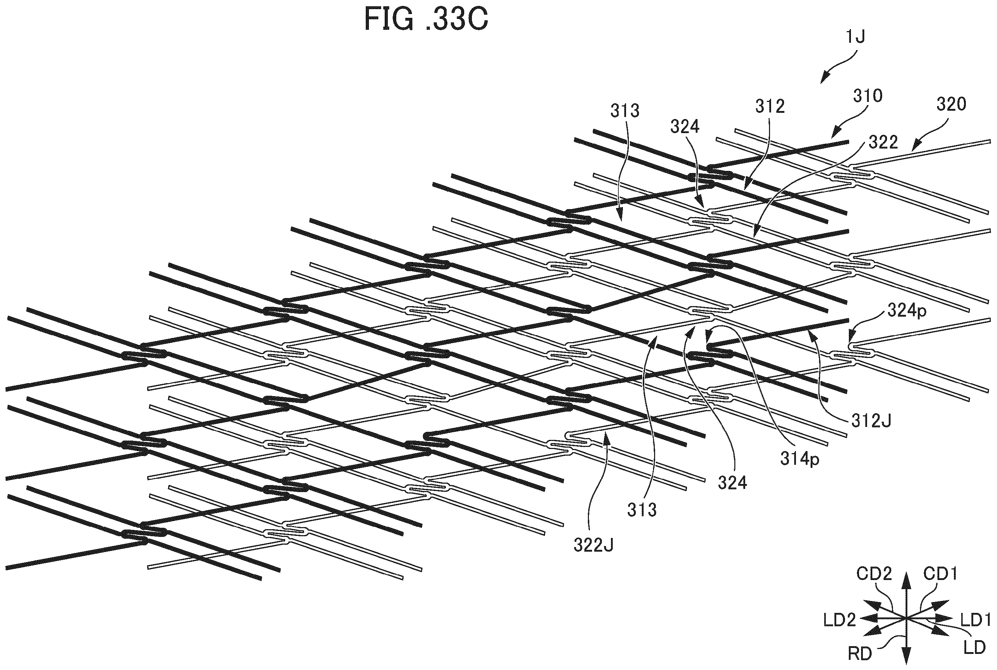

FIG. 33A is a development view showing a state in which a first stent body 310 of the fifteenth embodiment is virtually opened in a planar shape. FIG. 33B is a development view showing a state in which a second stent body 320 of the fifteenth embodiment is virtually opened in a planar shape. FIG. 33C is a development view showing a state in which the stent 1J of the fifteenth embodiment is virtually opened in a planar shape.

The first stent body 310 of the fifteenth embodiment is configured such that a plurality of outer cells 312 include outer cells 312J (described later) having a different configuration of a strut 311. As shown in FIG. 33A, the first stent body 310 of the fifteenth embodiment includes the outer cells 312 (hereinafter also referred to as the "outer cells 312J") each of which has no strut 311b of a pair of struts 311a to 311b arranged in the annular direction CD1. In the outer cell 312J, a raised portion 314p is provided at a portion where an intersection 314 not connected to the strut 311b and the strut 311 extending in the annular direction CD1 are connected to each other. The raised portion 314p protrudes to the distal side LD2, and therefore, when the stent 1H expanded in a blood vessel is resheathed in a catheter, contact between the raised portion 314p and an end portion of the catheter can be reduced. Note that the outer cells 312J may be arranged regularly or irregularly in the first stent body 310. Other configurations of the first stent body 310 of the fifteenth embodiment are the same as those of the fourth embodiment.

The second stent body 320 of the fifteenth embodiment is configured such that a plurality of inner cells 322 include inner cells 322J (described later) having a different configuration of a strut 321. As shown in FIG. 33B, the second stent body 320 of the fifteenth embodiment includes the inner cells 322 (hereinafter also referred to as the "inner cells 322J") each of which has no strut 321b of a pair of struts 321a to 321b arranged in the annular direction CD1. In the inner cell 322J, a raised portion 324p is provided at a portion where an intersection 324 not connected to the strut 321b and the strut 321 extending in the annular direction CD1 are connected to each other. The raised portion 324p protrudes to the distal side LD2, and therefore, when the stent 1H expanded in a blood vessel is resheathed in a catheter, contact between the raised portion 324p and an end portion of the catheter can be reduced. Note that the inner cells 322J may be arranged regularly or irregularly. Other configurations of the second stent body 320 of the fifteenth embodiment are the same as those of the fourth embodiment.

In the stent 1J of the fifteenth embodiment, the plurality of outer cells 312 (including 312J) forming the first stent body 310 and the plurality of inner cells 322 (including 322J) forming the second stent body 320 have the same size, shape, and arrangement, as one example. That is, in the fifteenth embodiment, the mesh pattern of the first stent body 310 shown in FIG. 33A and the mesh pattern of the second stent body 320 shown in FIG. 33B are substantially the same pattern. Note that the mesh pattern of the first stent body 310 and the mesh pattern of the second stent body 320 may be different from each other.

As shown in FIG. 33C, in the stent 1J of the fifteenth embodiment, the first stent body 310 and the second stent body 320 overlap with each other such that the second intersection 324 between the inner cells 322 (of the second stent body 320) is arranged in a hole 313 of the outer cell 312 (of the first stent body 310). Specifically, in a configuration in which the second intersection 324 between the inner cells 322 is arranged in the hole 313 of the outer cell 312 (312J), the first stent body 310 and the second stent body 320 overlap with each other such that one second intersection 324 between the inner cells 322 is arranged in one hole 313 of the outer cell 312 (312J).

In a case where each stent body is configured, as in the fifteenth embodiment, with the cell shape shown in FIGS. 33A and 33B, the mesh patterns of the stent bodies overlap with each other as described above so that the density of the mesh pattern is increased over the entire stent and the surface area of the stent 1J can be more increased. In the stent 1J of the fifteenth embodiment, the outer cells 312 (312J) and the inner cells 322 (322J) spread in the radial direction RD can be more flexibly bent as in the stent 1C of the fourth embodiment when the expanded stent 1J is bent substantially in a U-shape (see FIG. 7), and therefore, the shape followability of the stent 1J can be more enhanced.

In the stent 1J of the fifteenth embodiment, the raised portion 314p of the first stent body 310 and the raised portion 324p of the second stent body 320 protrude to the distal side LD2. Thus, when the stent 1J expanded in a blood vessel is resheathed in a catheter, contact among the raised portions 314p, 324p and an end portion of the catheter can be reduced. Thus, according to the stent 1J of the fifteenth embodiment, the stent 1J expanded in the blood vessel can be smoothly resheathed in the catheter.

### (Sixteenth Embodiment)

Next, a stent 1K of a sixteenth embodiment will be described. The stent 1K of the sixteenth embodiment is different from that of the fourth embodiment (see FIGS. 12A and 12B) in the cell shapes of first and second stent bodies. Other configurations of the stent 1K of the sixteenth embodiment are the same as those of the fourth embodiment. Thus, in the sixteenth embodiment, the entirety of the stent 1K is not shown in the figure. In description and drawings of the sixteenth embodiment, the same reference numerals are used to represent elements having functions similar to those of the fourth embodiment, and overlapping description thereof will be omitted as necessary. In the sixteenth embodiment, annular directions of cells being connected diagonally to a radial direction (circumferential direction) RD will be referred to as annular directions CD1, CD2. In the sixteenth embodiment, the "cell" is not limited to one configured such that struts 311 are arranged in a frame shape, and may include forms (e.g., an outer cell 312K and an inner cell 322K described later) in which struts 311 are not arranged in a frame shape.

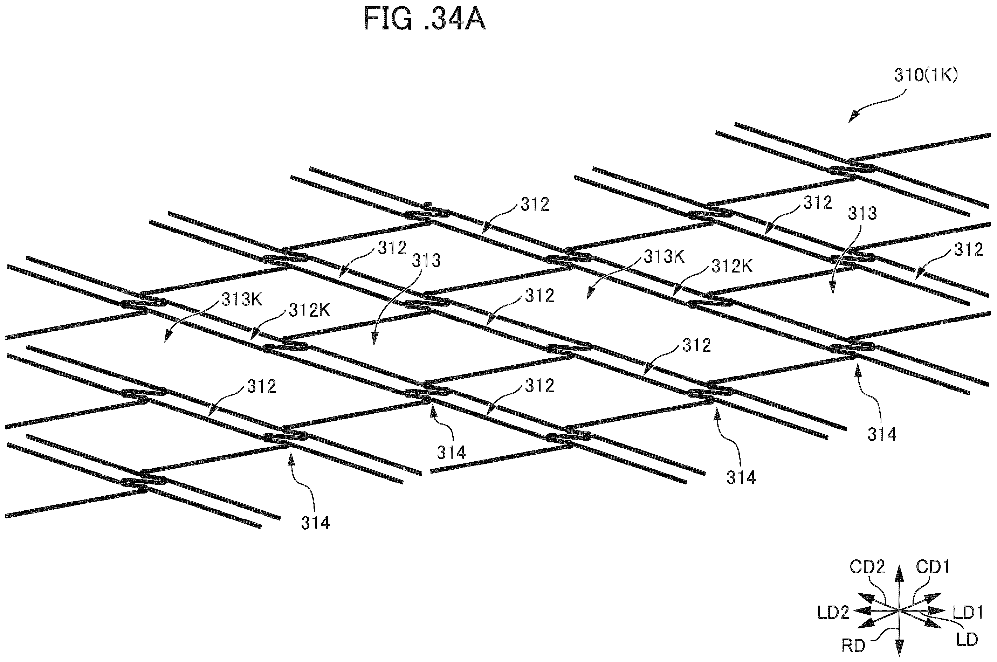

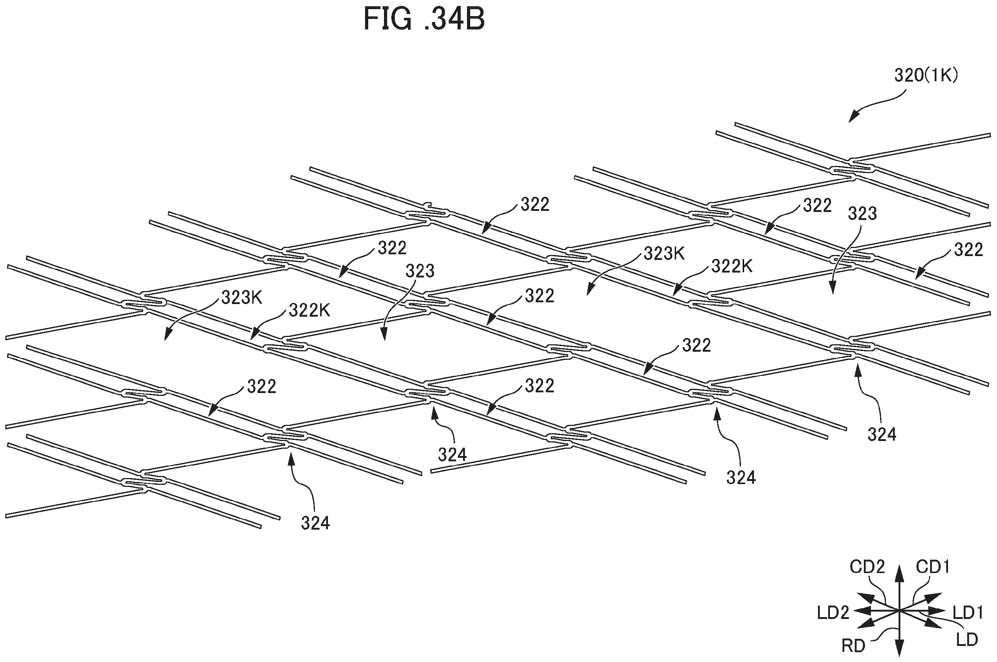

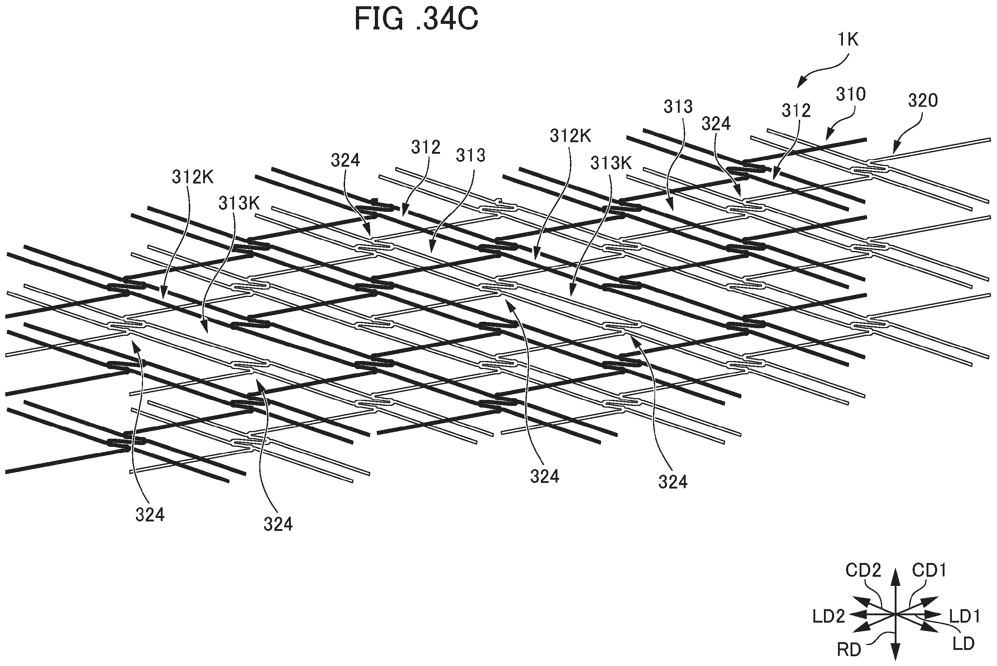

FIG. 34A is a development view showing a state in which a first stent body 310 of the sixteenth embodiment is virtually opened in a planar shape. FIG. 34B is a development view showing a state in which a second stent body 320 of the sixteenth embodiment is virtually opened in a planar shape. FIG. 34C is a development view showing a state in which the stent 1K of the sixteenth embodiment is virtually opened in a planar shape.

The first stent body 310 of the sixteenth embodiment is configured such that a plurality of outer cells 312 include the outer cells 312K (described later) having a different size. As shown in FIG. 34A, the first stent body 310 of the sixteenth embodiment includes the outer cells 312 (hereinafter also referred to as the "outer cells 312K") adjacent two of which in the annular direction CD2 has no common strut 311. A hole 313K of the outer cell 312K has a size twice as large as holes 313 of the other outer cells 312. Note that the outer cells 312K may be arranged regularly or irregularly in the first stent body 310. Other configurations of the first stent body 310 of the sixteenth embodiment are the same as those of the fourth embodiment.

The second stent body 320 of the sixteenth embodiment is configured such that a plurality of inner cells 322 include the inner cells 322K (described later) having a different size. As shown in FIG. 34B, the second stent body 320 of the sixteenth embodiment includes the inner cells 322 (hereinafter also referred to as the "inner cells 322K") adjacent two of which in the annular direction CD2 has no common strut 321. A hole 323K of the inner cell 322K has a size twice as large as holes 323 of the other inner cells 322. Note that the inner cells 322K may be arranged regularly or irregularly in the second stent body 320. Other configurations of the second stent body 320 of the sixteenth embodiment are the same as those of the fourth embodiment.

As shown in FIG. 34C, in the stent 1K of the sixteenth embodiment, the first stent body 310 and the second stent body 320 overlap with each other such that a second intersection 324 between the inner cells 322 (of the second stent body 320) is arranged in the hole 313 of the outer cell 312 (of the first stent body 310). Specifically, in a configuration in which the second intersection 324 between the inner cells 322 is arranged in the hole 313 of the outer cell 312, the first stent body 310 and the second stent body 320 overlap with each other such that one second intersection 324 between the inner cells 322 is arranged in one hole 313 of the outer cell 312. Since the first stent body 310 and the second stent body 320 overlap with each other as described above, the stent 1K is in such a form that two second intersections 324 between the inner cells 322 are arranged in the hole 313K of the outer cell 312K.

In a case where each stent body is configured, as in the sixteenth embodiment, with the cell shape shown in FIGS. 34A and 34B, the mesh patterns of the stent bodies overlap with each other as described above so that the density of the mesh pattern is increased over the entire stent and the surface area of the stent 1K can be more increased. In the stent 1K of the sixteenth embodiment, the outer cells 312 (312K) and the inner cells 322 (322K) spread in the radial direction RD can be more flexibly bent as in the stent 1C of the fourth embodiment when the expanded stent 1K is bent substantially in a U-shape (see FIG. 7), and therefore, the shape followability of the stent 1K can be more enhanced.

The embodiments of the present invention have been described above, but the present invention is not limited to the above-described embodiments and various modifications and changes can be made. These modifications and changes are also included in the scope of the present invention. The advantageous effects described in the embodiments are merely listed as most-suitable advantageous effects of the present invention, and the advantageous effects are not limited to those described in the embodiments. Note that the above-described embodiments and various modified or changed configurations may be combined as necessary, but detailed description thereof will be omitted.

The stent 1 of the first embodiment has the double-layer structure of the first stent body 10 and the second stent body 20, but is not limited to this structure. Another stent body may be further provided outside the first stent body 10 and/or inside the second stent body 20. The same also applies to the stents of the other embodiments.

In the stent 1 of the first embodiment, the surface(s) of the first stent body 10 and/or the second stent body 20 may be coated with a medical agent or a carbon-based material coating film, or be coated with metal or polymer having a high radiopacity. Examples of the medical agent may include a medical agent used for the same purpose as that of a drug-eluting stent (DES). Examples of the carbon-based material coating film may include an antithrombogenic inactive coating film such as diamond-like carbon (DLC). The same also applies to the stents of the other embodiments.

In the seventh to ninth embodiments, the connection portion of the first stent body and the connection portion of the second stent body may be provided at one location for the push wire 2, or the connection portions of the first stent body and the connection portions of the second stent body may be provided at three or more locations for the push wire 2. In the fourteenth to sixteenth embodiments, the positions of the intersections and struts omitted from the stent body are not limited to those of the illustrated examples. As long as the stent expanded in a blood vessel can be resheathed in a catheter, the positions of the intersections and struts omitted from the stent body can be selected as necessary.

Embodiments of the present invention will now be described in the form of numbered clauses:
1. A stent that is inserted into a catheter and pushed out of the catheter in a blood vessel to expand the blood vessel, comprising:
   a first stent body configured such that a plurality of first cells including struts arranged in a frame shape spreads in a circumferential direction and is continuously arranged in a center axis direction; and
   a second stent body configured such that a plurality of second cells including struts arranged in a frame shape spreads in a circumferential direction and is continuously arranged in a center axis direction and inserted into the first stent body,
   wherein in a state in which the second stent body is inserted into the first stent body, an intersection between the second cells is arranged in a hole of each first cell, and
   the first stent body and the second stent body are not coupled to each other in a radial direction.
2. The stent according to clause 1, wherein
   in the state in which the second stent body is inserted into the first stent body, the second stent body presses the first stent body outward in the radial direction.
3. The stent according to clause 1 or 2, wherein
   in a configuration in which the intersection between the second cells is arranged in the hole of each first cell, one intersection between the second cells is arranged in one hole of each first cell.
4. The stent according to any one of clauses 1 to 3, wherein
   in the state in which the second stent body is inserted into the first stent body, a percentage of a non-hole portion per unit surface area in a portion where the first stent body and the second stent body overlap with each other is 5 to 50%.
5. The stent according to any one of clauses 1 to 4, wherein
   each first cell includes, in an annular direction inclined with respect to the circumferential direction, a pair of first struts and one first strut arranged with a clearance from the pair of first struts, and
   each second cell includes, in an annular direction inclined with respect to the circumferential direction, a pair of second struts and one second strut arranged with a clearance from the pair of second struts.
6. The stent according to clause 5, wherein
   adjacent ones of the plurality of first cells are connected to each other at a substantially S-shaped first intersection in the annular direction inclined with respect to the circumferential direction, and
   adjacent ones of the plurality of second cells are connected to each other at a substantially S-shaped second intersection in the annular direction inclined with respect to the circumferential direction.
7. The stent according to clause 6, wherein
   the annular direction in which the plurality of first cells is connected at the first intersections and the annular direction in which the plurality of second cells is connected at the second intersections are symmetrical with respect to a line along the radial direction.
8. The stent according to any one of clauses 1 to 7, wherein
   a proximal side end portion of the first stent body and a proximal side end portion of the second stent body are connected at different positions in an axial direction of a push wire.
9. The stent according to any one of clauses 1 to 8, further comprising:
   a coating film between the first stent body and the second stent body.
10. The stent according to any one of clauses 1 to 9, wherein
   a strand having a high radiopacity is wound around at least one of the first stent body or the second stent body in a spiral shape.

### EXPLANATION OF REFERENCE NUMERALS

1, 1A, 1B, 1C, 1D, 1E, 1F, 1G, 1H, 1J, 1K Stent
2 Push Wire
3 Distal End Shaft
10, 110, 210, 310, 410, 510, 610 First Stent Body
20, 120, 220, 320, 420, 520, 620 Second Stent Body
12, 112, 212, 312 (312J, 312K), 412, 512, 612 Outer Cell
22, 122, 222, 322 (322J, 322K), 422, 522, 622 Inner Cell
11, 111, 211 (211a, 211b), 311 (311a, 311b), 411 (411a, 411b),
511 (511a, 511b), 611 Strut (Outer Cell)
21, 121, 221 (221a, 221b), 321 (321a, 321b), 421 (421a, 421b),
521 (521a, 521b), 621 Strut (Inner Cell)
13, 113, 213, 313 (313K), 413, 513, 613 Hole (Outer Cell)
23, 123, 223, 323 (323K), 423, 523, 623 Hole (Inner Cell)
14, 114 Intersecting Point (Outer Cell)
24, 124 Intersecting Point (Inner Cell)
214, 314, 614 Intersection (Outer Cell)
224, 324, 624 Intersection (Inner Cell)
314, 414, 514 First Intersection (Outer Cell)
324, 424, 524 Second Intersection (Inner Cell)
314p, 324p Raised Portion
30 Metal Wire
31 Strand with High Radiopacity
40 Coating Film

## Claims

1. A stent (1, 1A, 1B, 1C, 1D, 1E, 1F, 1G, 1H, 1J, 1K) that is inserted into a catheter and pushed out of the catheter in a blood vessel to expand the blood vessel, comprising:
a first stent body (10, 110, 210, 310, 410, 510, 610) configured such that a plurality of first cells (12, 112, 212, 312, 412, 512, 612) including struts (11, 111, 211, 311, 411, 511, 611) arranged in a frame shape spreads in a circumferential direction (OD) and is continuously arranged in a center axis direction (LD); and
a second stent body (20, 120, 220, 320, 420, 520, 620) configured such that a plurality of second cells (22, 122, 222, 322, 422, 522, 622) including struts (21, 121, 221, 321, 421, 521, 621) arranged in a frame shape spreads in a circumferential direction (OD) and is continuously arranged in a center axis direction (LD) and inserted into the first stent body (10, 110, 210, 310, 410, 510, 610),
wherein in a state in which the second stent body (20, 120, 220, 320, 420, 520, 620) is inserted into the first stent body (10, 110, 210, 310, 410, 510, 610), an intersection (14, 114, 214, 314, 414, 514, 614) between the second cells (22, 122, 222, 322, 422, 522, 622) is arranged in a hole (13, 113, 213, 313, 413, 513, 613) of each first cell (12, 112, 212, 312, 412, 512, 612),
wherein in a diameter-expanded state, the first stent body (10, 110, 210, 310, 410, 510, 610) and the second stent body (20, 120, 220, 320, 420, 520, 620) closely contact each other and are not coupled to each other in a radial direction (RD) and,
wherein a proximal side end portion of the first stent body (10, 110, 210, 310, 410, 510, 610) and a proximal side end portion of the second stent body (20, 120, 220, 320, 420, 520, 620) are connected to a push wire (2) undetachably.

2. The stent according to claim 1, wherein
in the state in which the second stent body is inserted into the first stent body (10, 110, 210, 310, 410, 510, 610), the second stent body (20, 120, 220, 320, 420, 520, 620) presses the first stent body (10, 110, 210, 310, 410, 510, 610) outward in the radial direction (RD).

3. The stent according to claim 1, wherein
in a configuration in which the intersection (24, 124, 224, 324, 424, 524, 624) between the second cells (22, 122, 222, 322, 422, 522, 622) is arranged in the hole (13, 113, 213, 313, 413, 513, 613) of each first cell (12, 112, 212, 312, 412, 512, 612), one intersection (24, 124, 224, 324, 424, 524, 624) between the second cells (22, 122, 222, 322, 422, 522, 622) is arranged in one hole (13, 113, 213, 313, 413, 513, 613) of each first cell (12, 112, 212, 312, 412, 512, 612).

4. The stent according to claim 1, wherein
in the state in which the second stent body (20, 120, 220, 320, 420, 520, 620) is inserted into the first stent body (10, 110, 210, 310, 410, 510, 610), a percentage of a non-hole portion per unit surface area in a portion where the first stent body (10, 110, 210, 310, 410, 510, 610) and the second stent body (20, 120, 220, 320, 420, 520, 620) overlap with each other is 5 to 50%.

5. The stent according to claim 1, wherein
each first cell (212, 312, 412, 512) includes, in an annular direction (CD1, CD2) inclined with respect to the circumferential direction (OD), a pair of first struts (211a-211b, 311a-311b, 411a-411b, 511a-511b) and one first strut (211, 311, 411, 511) arranged with a clearance from the pair of first struts (211a-211b, 311a-311b, 411a-411b, 511a-511b), and
each second cell (222, 322, 422, 522) includes, in an annular direction (CD1, CD2) inclined with respect to the circumferential direction (OD), a pair of second struts (221a-221b, 321a-321b, 421a-421b, 521a-521b) and one second strut (221, 321, 421, 521) arranged with a clearance from the pair of second struts (221a-221b, 321a-321b, 421a-421b, 521a-521b).

6. The stent according to claim 5, wherein
adjacent ones of the plurality of first cells (312, 412, 512) are connected to each other at a substantially S-shaped first intersection (314, 414, 514) in the annular direction (CD1, CD2) inclined with respect to the circumferential direction (OD), and
adjacent ones of the plurality of second cells (322, 422, 522) are connected to each other at a substantially S-shaped second intersection (324, 424, 524) in the annular direction (CD1, CD2) inclined with respect to the circumferential direction (OD).

7. The stent according to claim 6, wherein
the annular direction (CD1) in which the plurality of first cells (412) is connected at the first intersections (414) and the annular direction (CD2) in which the plurality of second cells (422) is connected at the second intersections (424) are symmetrical with respect to a line along the radial direction (RD).
